# EUROPEAN PATENT SPECIFICATION

(11) **EP 2 379 493 B1**
(45) Date of publication and mention of the grant of the patent: **14.10.2015**
(21) Application number: 09831324.0
(22) Date of filing: 11.12.2009
(51) Int. Cl.: C07C 337/08, A61K 38/08, C07D 295/135, A61K 31/28, A61K 51/04, C07F 1/08, A61K 31/402, A61K 51/06, C07F 3/06, A61K 31/47, A61P 35/00, C07F 5/02, A61K 31/4706, C07D 207/448, C07F 9/40

(54) **PROCESS FOR THE PREPARATION OF ASYMMETRICAL BIS(THIOSEMICARBAZONES)**
VERFAHREN ZUR HERSTELLUNG ASYMMETRISCHER BIS(THIOSEMICARBAZONE)
PROCÉDÉ DE PRÉPARATION DE BIS(THIOSEMICARBAZONES) ASYMÉTRIQUES

(30) Priority: 12.12.2008 AU 2008906411
(43) Date of publication of application: 26.10.2011
(73) Proprietor: The University of Melbourne, Parkville, Victoria 3010 (AU)
(72) Inventor: DONNELLY, Paul, Stephen, Brunswick East Victoria 3057 (AU); PATERSON, Brett, Michael, Flemington Victoria 3031 (AU)
(74) Representative: Osha Liang
(86) International application number: PCT/AU2009/001612
(87) International publication number: WO 2010/066010

(56) References cited:
- EP-B1- 0 024 464
- WO-A1-2008/061306
- WO-A2-2007/003944
- JP-A- 7 072 571
- LIM, J. K. ET AL.: 'Mixed Bis(thiosemicarbazone) Ligands for the Preparation of Copper Radiopharmaceuticals: Synthesis and Evaluation of Tetradentate Ligands Containing Two Dissimilar Thiosemicarbazone Functions.' J. MED. CHEM. vol. 40, no. 1, 1997, pages 132 - 136, XP055030121
- ACKERMAN, L. J. ET AL.: 'Synthesis and Evaluation of Copper Radiopharmaceuticals with Mixed Bis(thiosemicarbazone) Ligands.' NUCLEAR MEDICINE & BIOLOGY vol. 26, 1999, pages 551 - 554, XP027403205
- WINKELMANN, E. ET AL.: 'Anticoccidial Activity of Dithiosemicarbazones.' ARZNEIM.- FORSCH./DRUG RES. vol. 27, 1977, pages 950 - 967, XP009160332
- HOLLAND, J. P. ET AL.: 'Functionalized Bis(thiosemicarbazonato) Complexes of Zinc and Copper: Synthetic Platforms Toward Site-Specific Radiopharmaceuticals.' INORG. CHEM. vol. 46, no. 2, 2007, pages 465 - 485, XP055030122
- HOLLAND, J. P ET AL.: 'Synthesis, Radiolabelling and Confocal Fluorescence Microscopy of Styrene-Derivatised Bis(thiosemicarbazonato)zinc and -copper Complexes.' EUR. J. INORG. CHEM. 10 March 2008, pages 1985 - 1993, XP055030123
- MCPHERSON, D. W. ET AL.: 'Radiolabeling of proteins with radioisotopes of copper using p- carboxyalkylphenylglyoxal bis-(4N-methylthiosemicarbazone) (TSC) bifunctional chelates.' JOURNAL OF LABELLED COMPOUNDS AND RADIOPHARMACEUTICALS vol. 28, no. 8, 1990, pages 877 - 899, XP055030124
- DILANYAN, E. R. ET AL.: 'Antitumour activity of substituted methylglyoxal bisthiosemicarbazones and their copper(II) chelates.' PHARMACEUTICAL CHEMISTRY JOURNAL vol. 42, no. 9, September 2008, pages 504 - 506, XP055092627
- COATS, E. A. ET AL.: 'Comparative Analysis of the Cytotoxicity of Substituted [Phenylglyoxal bis(4-methyl-3-thiosemicarbazone)]copper(II ) Chelates. 2. Parabolic Correlations and Their Implications for Selective Toxicity.' J. MED. CHEM. vol. 21, no. 8, 1978, pages 804 - 809, XP055030125
- Andrew R. Cowley ET AL: "Bifunctional chelators for copper radiopharmaceuticals: the synthesis of [Cu(ATSM)?amino acid] and [Cu(ATSM)?octreotide] conjugates", Dalton Transactions, no. 2, 1 January 2007 (2007-01-01), page 209, XP055057998, ISSN: 1477-9226, DOI: 10.1039/b612142j

## Description

### FIELD

The present invention relates to a method of making asymmetrical bis(thiosemicarbazones), compounds useful as synthetic intermediates in the method, new bis(thiosemicarbazones) that can be readily accessed by use of the method and methods of treatment and imaging utilising some of the new bis(thiosemicarbazones).

### BACKGROUND

Bis(thiosemicarbazones) and their transition metal complexes are known to have a broad range of pharmacological activity. For example, different derivatives have been identified which possess anti-cancer properties, act as superoxide dismutase-like reactive oxygen species scavengers, possess anti-bacterial activity and more recently as therapeutic agents for neurodegenerative diseases. In addition these bis(thiosemicarbazone) ligands have shown considerable potential as vehicles for the delivery of radioactive copper isotopes in the development of copper radiopharmaceuticals.

WO2008/061306 and WO2007/003944 disclose bis(thiosemicarbazone) metal complexes for imaging and therapy.

In the area of copper radiopharmaceuticals there are several radionuclides of copper that have the potential to be used in diagnostic imaging agents or in radiotherapy. Copper-60, copper-61, copper-62 and copper-64 are all positron emitters with potential applications in positron emission tomography (PET). In all cases, the form in which the radionuclide is administered is crucial and it is essential that the radionuclide be delivered selectively to the target area. This can be achieved by the formation of a copper coordination complex where the biodistribution is determined by several factors such as charge, shape, lipophilicity and redox chemistry.

Site specific imaging and/or treatment can be attained by tethering a biologically active molecule (which selectively binds to certain receptor sites *in vivo*) to the complex. A pendant arm, such as a carboxylate functional group, can be used to form an amide linkage with biomolecules *via* established peptide coupling methodology to a terminal amine on the biologically active molecule. In such cases the formal charge, size and other factors associated with the metal complex can alter its bio-distribution and it is essential that the bifunctional chelate is sufficiently stable *in vivo.*

*Bis*(thiosemicarbazone) ligands derived from 1,2-diones form stable, neutral, low molecular weight, planar complexes with copper(II) and have been successfully used as chelators for radiocopper. For example [Cu(PTSM)] has been investigated as a perfusion imaging agent whereas [Cu(ATSM)] has been shown to be a successful hypoxia tracer due to its selective retention in hypoxic tissue.

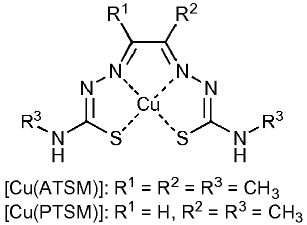

*Bis*(thiosemicarbazone) ligands provide an N₂S₂ chelate system for copper. The mixture of hard nitrogen donor and soft sulfur donor atoms provides a hybrid system that is capable of forming stable Cu(II) and Cu(I) complexes. This behaviour and the fact that they form formally charge neutral complexes means that *bis*(thiosemicarbazones) have potential advantages as radiocopper chelators over tetra-aza macrocyclic systems based on TETA (1,4,8,11-tetraazacyclotetradecane-*N,N,'N",N"'*-tetraacetic acid).

Accordingly there is significant interest in the development of *bis*(thiosemicarbazone) ligands based on this core structural backbone as they would be expected to be relatively stable as discussed above. A difficulty with ligands of this type is that in order to be used in targeting applications there is the requirement that the ligand contain a moiety that is, or can be, linked to a molecular recognition moiety. In general in order to achieve this there is a requirement to synthesize asymmetric *bis*(thiosemicarbazone) ligands.

This presents difficulty as the present methodology for the synthesis of asymmetric *bis*(thiosemicarbazone) ligands typically involves a stepwise reaction of the appropriate dione with a suitably functionalised thiosemicarbazide to form the mono-adduct followed by reaction of the material thus formed with another suitably functionalised thiosemicarbazide.

There are a number of difficulties with this approach. Firstly, in the first step of the process the mono-adduct is rarely formed exclusively and so separation of the desired product from the reaction mixture which typically contains starting material, the mono adduct and the bis adduct is required. This reduces the overall yield in the process and increases the prospect of undesirable impurities being incorporated into the final product which is clearly undesirable in a product destined for a pharmaceutical use.

Accordingly this process is somewhat cumbersome and undesirable from a commercial manufacturing standpoint. In addition, in many instances the thiosemicarbazide required to provide the desired functionality in the final product is not readily available or easy to synthesize from commercially available starting materials. This therefore limits the degree of flexibility in the final ligands that can be readily produced using these known techniques. As such there is a clear need to provide an improved method for the synthesis of asymmetric *bis*(thiosemicarbazone) ligands.

The present applicants have identified that a versatile and efficient method of synthesis of a wide variety of asymmetric *bis*(thiosemicarbazone) ligands can be achieved by taking advantage of the finding that if a ligand of this type is produced with secondary and tertiary nitrogen atoms at the respective terminal ends the tertiary nitrogen can take part in a selective trans-amination reaction to introduce a wide variety of substituents at this position on the ligand.

### SUMMARY

In one aspect the present invention provides a method of making a compound of the formula (I): 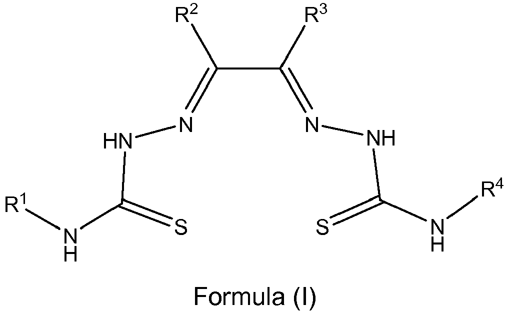 wherein
R¹ is selected from the group consisting of C₁-C₁₂alkyl, C₂-C₁₂alkenyl, C₂-C₁₂alkynyl, C₂-C₁₂heteroalkyl, C₃-C₁₂cycloalkyl, C₂-C₁₂heterocycloalkyl, C₆-C₁₈aryl, and C₁-C₁₈heteroaryl;
R⁴ is selected from the group consisting of optionally substituted C₁-C₁₂alkyl, optionally substituted C₂-C₁₂alkenyl, optionally substituted C₂-C₁₂alkynyl, optionally substituted C₂-C₁₂heteroalkyl, optionally substituted C₃-C₉cycloalkyl, optionally substituted C₃-C₉cycloalkenyl, optionally substituted C₂-C₁₂heterocycloalkyl, optionally substituted C₂-C₁₂heterocycloalkenyl, optionally substituted C₆-C₁₈aryl, optionally substituted C₁-C₁₈heteroaryl, optionally substituted C₃-C₉cycloalkylC₁-C₁₂alkyl, C₂-C₁₂heterocycloalkylC₁-C₁₂alkyl, optionally substituted C₆-C₁₈arylC₁-C₁₂alkyl, optionally substituted C₁-C₁₈heteroarylC₁-Cₗ₂ alkyl, optionally substituted C₆-C₁₈arylC₂-C₁₂heteroalkyl, optionally substituted C₃-C₉cycloalkylC₂-C₁₂heteroalkyl, optionally substituted C₆-C₁₈arylC₂-C₁₂heteroalkyl, optionally substituted C₂-C₁₂heterocycloalkylC₂-C₁₂heteroalkyl, and optionally substituted C₁-C₁₈heteroaryl C₂-C₁₂heteroalkyl such that R¹ and R⁴ are not the same;
R² and R³ are each independently selected from the group consisting of: H, and C₁-C₁₂alkyl
the method comprising reacting a compound of formula (II) 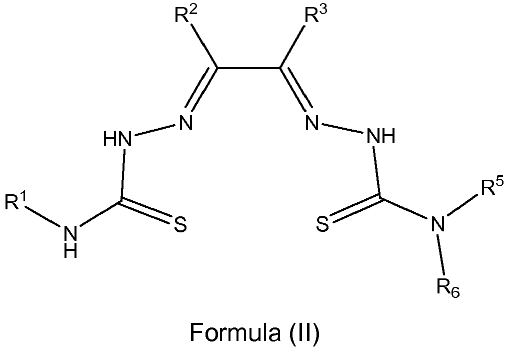
wherein R¹, R², and R³ are as defined above and R⁵ and R⁶ are each independently C₁-C₁₂alkyl;
with a primary amine of formula (III) NH₂R⁴;
wherein R⁴ is as defined above.

In some embodiments R¹ is methyl.

In some embodiments of the method of the invention R² and R³ are methyl.

In some embodiments R⁵ and R⁶ are each independently selected from the group consisting of methyl, ethyl, isopropyl, propyl, 2-methyl-propyl, 1-ethyl-propyl, 3,3-dimethyl-propyl, butyl, isobutyl, 3,3-dimethyl-butyl, 2-ethyl-butyl, pentyl, and hexyl. In some embodiments R⁵ and R⁶ are methyl.

Accordingly in some embodiments of the methods of the invention there is provided a method of synthesis of a compound of formula (Ia) 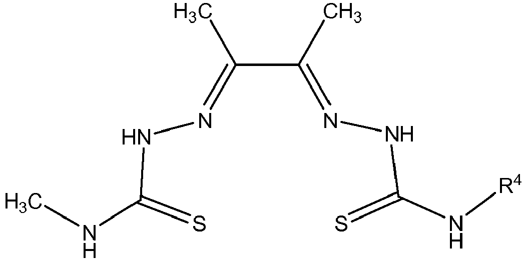 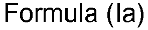 wherein
R⁴ is selected from the group consisting of optionally substituted C₁-C₁₂alkyl, optionally substituted C₂-C₁₂alkenyl, optionally substituted C₂-C₁₂alkynyl, optionally substituted C₂-C₁₂heteroalkyl, optionally substituted C₃-C₉cycloalkyl, optionally substituted C₃-C₉cycloalkenyl, optionally substituted C₂-C₁₂heterocycloalkyl, optionally substituted C₂-C₁₂heterocycloalkenyl, optionally substituted C₆-C₁₈aryl, optionally substituted C₁-C₁₈heteroaryl, optionally substituted C₃-C₉cycloalkylC₁-C₁₂alkyl, C₂-C₁₂heterocycloalkylC₁-C₁₂alkyl, optionally substituted C₆-C₁₈arylC₁-C₁₂alkyl, optionally substituted C₁-C₁₈heteroarylC₁-C₁₂ alkyl, optionally substituted C₆-C₁₈arylC₂-C₁₂heteroalkyl, optionally substituted C₃-C₉cycloalkylC₂-C₁₂heteroalkyl, optionally substituted C₆-C₁₈arylC₂-C₁₂heteroalkyl, optionally substituted C₂-C₁₂heterocycloalkylC₂-C₁₂heteroalkyl, and optionally substituted C₁-C₁₈heteroaryl C₂-C₁₂heteroalkyl;
the method comprising reacting a compound of formula (IIa) 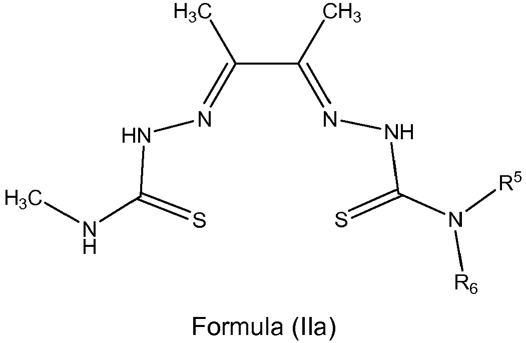
wherein R⁵ and R⁶ are each independently C₁-C₁₂alkyl with a primary amine of formula NH₂R⁴.

The methods of the invention may be used to incorporate a wide range of R⁴ groups as in principle any primary amine may be used in the methods of the invention. The only requirement is that the amine be suitably reactive to displace the secondary amine on the starting material.

In some embodiments R⁴ is a group of the formula:

-X-Y

wherein X is selected from the group consisting of:
(a) a bond
(b) -(CH₂)ₘCO₂-
(c) -(CH₂)ₘCO-
(d) -(CH₂)ₘSO₃-
(e) -(CH₂)ₘSO₂-
(f) -(CH₂)ₘR⁸-
(g) -(CH₂)ₘCHR⁹R¹⁰;
(h) -(CH₂)ₘNHCO₂-
(i) -(CH₂)ₘNH-
(j) -(CH₂)ₘNR⁹-
(k) -(CH₂)ₘNHSO₂-
(l) -(CH₂)ₘSO₂-
(m) -(CH₂)ₘSO₃-
(n) -(CH₂)ₘR⁸-
(o) -(CH₂)ₘCHR⁹R¹⁰;
(p) -((CH₂)ₓO)_{y}-;
(q) -((CH₂)ₓNR₁₁)_{y}-;
wherein
m is an integer selected from the group consisting of 0, 1, 2, 3, 4, 5, 6, 7, 8, 9, and 10;
each x is independently an integer selected from the group consisting of 0, 1, 2, 3, 4, 5, 6, 7, 8, 9, and 10;
y is an integer selected from the group consisting of 0, 1, 2, 3, 4, 5, 6, 7, 8, 9, and 10;
R⁸ is selected from the group consisting of optionally substituted C₆-C₁₈aryl, and optionally substituted C₁-C₁₈heteroaryl,
each R⁹ and R¹⁰ is independently selected from the group consisting of CO₂H, optionally substituted C₁-C₁₂alkyl, and optionally substituted C₂-C₁₂heteroalkyl;
R¹¹ is independently selected from the group consisting of H, optionally substituted C₁-C₁₂alkyl, optionally substituted C₂-C₁₂heteroalkyl and a nitrogen protecting group;

Y is selected from the group consisting of H, optionally substituted C₁-C₁₂alkyl, optionally substituted C₂-C₁₂alkenyl, optionally substituted C₂-C₁₂alkynyl, optionally substituted C₂-C₁₂heteroalkyl, optionally substituted C₃-C₉cycloalkyl, optionally substituted C₃-C₉cycloalkenyl, optionally substituted C₂-C₁₂heterocycloalkyl, optionally substituted C₂-C₁₂heterocycloalkenyl, optionally substituted C₆-C₁₈aryl, optionally substituted C₁-C₁₈heteroaryl, optionally substituted C₃-C₉cycloalkylC₁-C₁₂alkyl, C₂-C₁₂heterocycloalkylC₁-C₁₂alkyl, optionally substituted C₆-C₁₈arylC₁-C₁₂alkyl, optionally substituted C₁-C₁₈heteroarylC₁-C₁₂ alkyl, optionally substituted C₆-C₁₈arylC₂-C₁₂heteroalkyl, optionally substituted C₃-C₉cycloalkylC₂-C₁₂heteroalkyl, optionally substituted C₆-C₁₈arylC₂-C₁₂heteroalkyl, optionally substituted C₂-C₁₂heterocycloalkylC₂-C₁₂heteroalkyl, optionally substituted C₁-C₁₈heteroaryl C₂-C₁₂heteroalkyl, a peptide, a protein and a molecular recognition moiety.

In the methods of the invention the X moiety serves as a linking moiety that ultimately serves to act as a spacer between the ligand which can be bound to a metal (which may be a radionuclide) and either the point of attachment of a molecular recognition moiety or the molecular recognition moiety *per se.* As such whilst it is desirable that there be a certain degree of separation between the two in order to ensure that the two entities do not interfere with each other's activity it is also important that the two are not so far removed such that the radionuclide is not effectively delivered to its site of operation.

In some embodiments X is a linking moiety having from 1 to 10 atoms in the normal chain. In some embodiments X is a linking moiety having from 1 to 8 atoms in the normal chain. In some embodiments X has 8 atoms in the normal chain. In some embodiments X has 7 atoms in the normal chain. In some embodiments X has 6 atoms in the normal chain. In some embodiments X has 5 atoms in the normal chain. In some embodiments X has 4 atoms in the normal chain. In some embodiments X has 3 atoms in the normal chain. In some embodiments X has 2 atoms in the normal chain. In some embodiments X has 1 atom in the normal chain.

In some embodiments X is selected from the group consisting of:
(a) -(CH₂)ₘCO₂-;
(b) -(CH₂)ₘCO-;
(c) -(CH₂)ₘSO₃-;
(d) -(CH₂)ₘSO₂-;
(e) -(CH₂)ₘR⁸-; and
(f) -(CH₂)ₘCHR⁹R¹⁰;
wherein m, R⁸, R⁹ and R¹⁰ are as defined above.

In some embodiments m is 0. In some embodiments m is 1. In some embodiments m is 2. In some embodiments m is 3. In some embodiments m is 4. In some embodiments m is 5. In some embodiments m is 6. In some embodiments m is 7. In some embodiments m is 8. In some embodiments m is 9. In some embodiments m is 10.

In some embodiments x is 0. In some embodiments x is 1. In some embodiments x is 2. In some embodiments x is 3. In some embodiments x is 4. In some embodiments x is 5. In some embodiments x is 6. In some embodiments m is 7. In some embodiments x is 8. In some embodiments x is 9. In some embodiments x is 10.

In some embodiments y is 0. In some embodiments y is 1. In some embodiments y is 2. In some embodiments y is 3. In some embodiments y is 4. In some embodiments y is 5. In some embodiments y is 6. In some embodiments y is 7. In some embodiments y is 8. In some embodiments y is 9. In some embodiments y is 10.

In some embodiments R⁸ is phenyl.

In some embodiments R⁹ and R¹⁰ are independently selected from the group consisting of CO₂H and methyl.

In some embodiments R¹¹ is selected from the group consisting of H and a nitrogen protecting group.

In some embodiments Y is H or a molecular recognition moiety selected from the group consisting of antibodies, proteins, peptides, carbohydrates, nucleic acids, oligonucleotides, oligosaccharides and liposomes.

In some embodiments of the method Y is a molecular recognition moiety. In some embodiments of the method Y is H.

In some embodiments X and Y are chosen such that the primary amine NH₂R⁴ is an amino acid group wherein the NH₂ group is the N-terminal portion of the amino acid group and R⁴ is the remainder of the amino acid group (or a protected form thereof). In essence this places an amino acid, or peptide on this side of the molecule.

The present invention also provides asymmetrical complexes that may be made by the method of synthesis of the invention as described above.

These and other features of the present teachings are set forth herein.

### DETAILED DESCRIPTION

In this specification a number of terms are used which are well known to a skilled addressee. Nevertheless for the purposes of clarity a number of terms will be defined.

As used herein, the term "unsubstituted" means that there is no substituent or that the only substituents are hydrogen.

The term "non-hydrogen substituent group" means any substituent that is not hydrogen. Exemplary non-hydrogen substituent groups include halogen, -CN, -NO₂,-CF₃, -OCF₃, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, cycloalkylalkyl, heterocycloalkylalkyl, heteroarylalkyl, arylalkyl, cycloalkylalkenyl, heterocycloalkylalkenyl, arylalkenyl, heteroarylalkenyl, cycloalkylheteroalkyl, heterocycloalkylheteroalkyl, arylheteroalkyl, heteroarylheteroalkyl, hydroxy, hydroxyalkyl, alkyloxy, alkyloxyalkyl, alkyloxycycloalkyl, alkyloxyheterocycloalkyl, alkyloxyaryl, alkyloxyheteroaryl, alkyloxycarbonyl, alkylaminocarbonyl, alkenyloxy, alkynyloxy, cycloalkyloxy, cycloalkenyloxy, heterocycloalkyloxy, heterocycloalkenyloxy, aryloxy, phenoxy, benzyloxy, heteroaryloxy, arylalkyloxy, alkylamino, acylamino, aminoalkyl, arylamino, sulfonylamino, sulfinylamino, sulfonyl, alkylsulfonyl, arylsulfonyl, aminosulfonyl, sulfinyl, alkylsulfinyl, arylsulfinyl, aminosulfinylaminoalkyl, -C(=O)OH, -C(=O)R^{a}, -C(=O)OR^{a}, C(=O)NR^{a}R^{b}, C(=NOH)R^{a}, C(=NR^{a})NR^{b}R^{c}, NR^{a}R^{b}, NR^{a}C(=O)R^{b}, NR^{a}C(=O)OR^{b}, NR^{a}C(=O)NR^{b}R^{c}, NR^{a}C(=NR^{b})NR^{c}R^{d}, NR^{a}SO₂R^{b}, -SR^{a}, SO₂NR^{a}R^{b}, -OR^{a}, OC(=O)NR^{a}R^{b}, OC(=O)R^{a} and acyl,
wherein R^{a}, R^{b}, R^{c} and R^{d} are each independently selected from the group consisting of H, C₁-C₁₂alkyl, C₁-C₁₂haloalkyl, C₂-C₁₂alkenyl, C₂-C₁₂alkynyl, C₂-C₁₀ heteroalkyl, C₃-C₁₂cycloalkyl, C₃-C₁₂cycloalkenyl, C₂-C₁₂heterocycloalkyl, C₂-C₁₂ heterocycloalkenyl, C₆-C₁₈aryl, C₁-C₁₈heteroaryl, and acyl, or any two or more of R^{a}, R^{b}, R^{c} and R^{d}, when taken together with the atoms to which they are attached form a heterocyclic ring system with 3 to 12 ring atoms.

Another suitable non-hydrogen substituent group is a group of the formula:

-X-Y

wherein X is selected from the group consisting of:
(a) a bond
(b) -(CH₂)ₘCO₂-
(c) -(CH₂)ₘCO-
(d) -(CH₂)ₘSO₃-
(e) -(CH₂)ₘSO₂-
(f) -(CH₂)ₘR⁸-
(g) -(CH₂)ₘCHR⁹R¹⁰;
(h) -(CH₂)ₘNHCO₂-
(i) -(CH₂)ₘNH-
(j) -(CH₂)ₘNR⁹-
(k) -(CH₂)ₘNHSO₂-
(l) -(CH₂)ₘSO₂-
(m) -(CH₂)ₘSO₃-
(n) -(CH₂)ₘR⁸-
(o) -(CH₂)ₘCHR⁹R¹⁰;
(p) -((CH₂)ₓO)_{y}-;
(q) -((CH₂)ₓNR₁₁)_{y}-;
wherein
m is an integer selected from the group consisting of 0, 1, 2, 3, 4, 5, 6, 7, 8, 9, and 10;
each x is independently an integer selected from the group consisting of 0, 1, 2, 3, 4, 5, 6, 7, 8, 9, and 10;
y is an integer selected from the group consisting of 0, 1, 2, 3, 4, 5, 6, 7, 8, 9, and 10;
R⁸ is selected from the group consisting of optionally substituted C₆-C₁₈aryl, and optionally substituted C₁-C₁₈heteroaryl,
each R⁹ and R¹⁰ is independently selected from the group consisting of CO₂H, optionally substituted C₁-C₁₂alkyl, and optionally substituted C₂-C₁₂heteroalkyl;
R¹¹ is independently selected from the group consisting of H, optionally substituted C₁-C₁₂alkyl, optionally substituted C₂-C₁₂heteroalkyl and a nitrogen protecting group;
Y is selected from the group consisting of H, optionally substituted C₁-C₁₂alkyl, optionally substituted C₂-C₁₂alkenyl, optionally substituted C₂-C₁₂alkynyl, optionally substituted C₂-C₁₂heteroalkyl, optionally substituted C₃-C₉cycloalkyl, optionally substituted C₃-C₉cycloalkenyl, optionally substituted C₂-C₁₂heterocycloalkyl, optionally substituted C₂-C₁₂heterocycloalkenyl, optionally substituted C₆-C₁₈aryl, optionally substituted C₁-C₁₈heteroaryl, optionally substituted C₃-C₉cycloalkylC₁-C₁₂alkyl, C₂-C₁₂heterocycloalkylC₁-C₁₂alkyl, optionally substituted C₆-C₁₈arylC₁-C₁₂alkyl, optionally substituted C₁-C₁₈heteroarylC₁-C₁₂ alkyl, optionally substituted C₆-C₁₈arylC₂-C₁₂heteroalkyl, optionally substituted C₃-C₉cycloalkylC₂-C₁₂heteroalkyl, optionally substituted C₆-C₁₈arylC₂-C₁₂heteroalkyl, optionally substituted C₂-C₁₂heterocycloalkylC₂-C₁₂heteroalkyl, optionally substituted C₁-C₁₈heteroaryl C₂-C₁₂heteroalkyl, a peptide, a protein and a molecular recognition moiety.

The term "optionally substituted" as used throughout the specification denotes that the group may or may not be further substituted or fused (so as to form a condensed polycyclic system), with one or more non-hydrogen substituent groups as defined above.

In some embodiments each optional substituent is independently selected from the group consisting of: halogen, =O, =S, -CN, -NO₂, -CF₃, -OCF₃, alkyl, alkenyl, alkynyl, haloalkyl, haloalkenyl, haloalkynyl, heteroalkyl, cycloalkyl, cycloalkenyl, heterocycloalkyl, heterocycloalkenyl, aryl, heteroaryl, hydroxy, hydroxyalkyl, alkyloxy, alkyloxyalkyl, alkyloxyaryl, alkyloxyheteroaryl, alkenyloxy, alkynyloxy, cycloalkyloxy, cycloalkenyloxy, heterocycloalkyloxy, heterocycloalkenyloxy, aryloxy, heteroaryloxy, arylalkyl, heteroarylalkyl, arylalkyloxy, amino, alkylamino, acylamino, aminoalkyl, arylamino, sulfonyl, alkylsulfonyl, arylsulfonyl, aminosulfonyl, aminoalkyl, -COOH, -SH, and acyl.

Examples of particularly suitable optional substituents include F, Cl, Br, I, CH₃, CH₂CH₃, OH, OCH₃, CF₃, OCF₃, NO₂, NH₂, and CN.

"Alkenyl" as a group or part of a group denotes an aliphatic hydrocarbon group containing at least one carbon-carbon double bond and which may be straight or branched preferably having 2-12 carbon atoms, more preferably 2-10 carbon atoms, most preferably 2-6 carbon atoms, in the normal chain. The group may contain a plurality of double bonds in the normal chain and the orientation about each is independently E or Z. Exemplary alkenyl groups include, but are not limited to, ethenyl, propenyl, butenyl, pentenyl, hexenyl, heptenyl, octenyl and nonenyl. The group may be a terminal group or a bridging group.

"Alkyl" as a group or part of a group refers to a straight or branched aliphatic hydrocarbon group, preferably a C₁-C₁₂ alkyl, more preferably a C₁-C₁₀ alkyl, most preferably C₁-C₆ unless otherwise noted. Examples of suitable straight and branched C₁-C₆ alkyl substituents include methyl, ethyl, n-propyl, 2-propyl, n-butyl, sec-butyl, t-butyl, hexyl, and the like. The group may be a terminal group or a bridging group.

"Alkynyl" as a group or part of a group means an aliphatic hydrocarbon group containing a carbon-carbon triple bond and which may be straight or branched preferably having from 2-12 carbon atoms, more preferably 2-10 carbon atoms, more preferably 2-6 carbon atoms in the normal chain. Exemplary structures include, but are not limited to, ethynyl and propynyl. The group may be a terminal group or a bridging group.

"Aryl" as a group or part of a group denotes (i) an optionally substituted monocyclic, or fused polycyclic, aromatic carbocycle (ring structure having ring atoms that are all carbon) preferably having from 5 to 12 atoms per ring. Examples of aryl groups include phenyl, naphthyl, and the like; (ii) an optionally substituted partially saturated bicyclic aromatic carbocyclic moiety in which a phenyl and a C₅₋₇ cycloalkyl or C₅₋₇ cycloalkenyl group are fused together to form a cyclic structure, such as tetrahydronaphthyl, indenyl or indanyl. The group may be a terminal group or a bridging group. Typically an aryl group is a C₆-C₁₈ aryl group.

"Arylalkyl" means an aryl-alkyl- group in which the aryl and alkyl moieties are as defined herein. Preferred arylalkyl groups contain a C₁₋₅alkyl moiety. Exemplary arylalkyl groups include benzyl, phenethyl, 1-naphthalenemethyl and 2-naphthalenemethyl. The group may be a terminal group or a bridging group. If the group is a terminal group it is bonded to the remainder of the molecule through the alkyl group.

"Arylheteroalkyl" means an aryl-heteroalkyl- group in which the aryl and heteroalkyl moieties are as defined herein. The group may be a terminal group or a bridging group. If the group is a terminal group it is bonded to the remainder of the molecule through the heteroalkyl group.

"Cycloalkyl" refers to a saturated monocyclic or fused or spiro polycyclic, carbocycle preferably containing from 3 to 9 carbons per ring, such as cyclopropyl, cyclobutyl, cyclopentyl, cyclohexyl and the like, unless otherwise specified. It includes monocyclic systems such as cyclopropyl and cyclohexyl, bicyclic systems such as decalin, and polycyclic systems such as adamantane. A cycloalkyl group typically is a C₃-C₉ cycloalkyl group. The group may be a terminal group or a bridging group.

"Cycloalkylalkyl" means a cycloalkyl-alkyl- group in which the cycloalkyl and alkyl moieties are as defined herein. Exemplary monocycloalkylalkyl groups include cyclopropylmethyl, cyclopentylmethyl, cyclohexylmethyl and cycloheptylmethyl. The group may be a terminal group or a bridging group. If the group is a terminal group it is bonded to the remainder of the molecule through the alkyl group.

"Cycloalkenyl" means a non-aromatic monocyclic or multicyclic ring system containing at least one carbon-carbon double bond and preferably having from 5-10 carbon atoms per ring. Exemplary monocyclic cycloalkenyl rings include cyclopentenyl, cyclohexenyl or cycloheptenyl. The cycloalkenyl group may be substituted by one or more substituent groups. A cycloalkenyl group typically is a C₃-C₁₂ alkenyl group. The group may be a terminal group or a bridging group.

"Cycloalkylheteroalkyl" means a cycloalkyl-heteroalkyl- group in which the cycloalkyl and heteroalkyl moieties are as defined herein. The group may be a terminal group or a bridging group. If the group is a terminal group it is bonded to the remainder of the molecule through the heteroalkyl group.

"Heteroalkyl" refers to a straight- or branched-chain alkyl group preferably having from 2 to 12 carbons, more preferably 2 to 6 carbons in the chain, in which one or more of the carbon atoms (and any associated hydrogen atoms) are each independently replaced by a heteroatomic group selected from S, O, P and NR' where R' is selected from the group consisting of H, optionally substituted C₁-C₁₂alkyl, optionally substituted C₃-C₁₂cycloalkyl, optionally substituted C₆-C₁₈aryl, and optionally substituted C₁-C₁₈heteroaryl. Exemplary heteroalkyls include alkyl ethers, secondary and tertiary alkyl amines, amides, alkyl sulfides, and the like. Examples of heteroalkyl also include hydroxyC₁-C₆alkyl, C₁-C₆alkyloxyC₁-C₆alkyl, aminoC₁-C₆alkyl, C₁-C₆alkylaminoC₁-C₆alkyl, and di(C₁-C₆alkyl)aminoC₁-C₆alkyl. The group may be a terminal group or a bridging group.

"Heteroaryl" either alone or part of a group refers to groups containing an aromatic ring (preferably a 5 or 6 membered aromatic ring) having one or more heteroatoms as ring atoms in the aromatic ring with the remainder of the ring atoms being carbon atoms. Suitable heteroatoms include nitrogen, oxygen and sulphur. Examples of heteroaryl include thiophene, benzothiophene, benzofuran, benzimidazole, benzoxazole, benzothiazole, benzisothiazole, naphtho[2,3-b]thiophene, furan, isoindolizine, xantholene, phenoxatine, pyrrole, imidazole, pyrazole, pyridine, pyrazine, pyrimidine, pyridazine, tetrazole, indole, isoindole, 1H-indazole, purine, quinoline, isoquinoline, phthalazine, naphthyridine, quinoxaline, cinnoline, carbazole, phenanthridine, acridine, phenazine, thiazole, isothiazole, phenothiazine, oxazole, isooxazole, furazane, phenoxazine, 2-, 3- or 4- pyridyl, 2-, 3-, 4-, 5-, or 8- quinolyl, 1-, 3-, 4-, or 5- isoquinolinyl 1-, 2-, or 3- indolyl, and 2-, or 3-thienyl. A heteroaryl group is typically a C₁-C₁₈ heteroaryl group. The group may be a terminal group or a bridging group.

"Heteroarylalkyl" means a heteroaryl-alkyl group in which the heteroaryl and alkyl moieties are as defined herein. Preferred heteroarylalkyl groups contain a lower alkyl moiety. Exemplary heteroarylalkyl groups include pyridylmethyl. The group may be a terminal group or a bridging group. If the group is a terminal group it is bonded to the remainder of the molecule through the alkyl group.

"Heteroarylheteroalkyl" means a heteroaryl-heteroalkyl- group in which the heteroaryl and heteroalkyl moieties are as defined herein. The group may be a terminal group or a bridging group. If the group is a terminal group it is bonded to the remainder of the molecule through the heteroalkyl group.

"Heterocycloalkyl" refers to a saturated monocyclic, bicyclic, or polycyclic ring containing at least one heteroatom selected from nitrogen, sulfur, oxygen, preferably from 1 to 3 heteroatoms in at least one ring. Each ring is preferably from 3 to 10 membered, more preferably 4 to 7 membered. Examples of suitable heterocycloalkyl substituents include pyrrolidyl, tetrahydrofuryl, tetrahydrothiofuranyl, piperidyl, piperazyl, tetrahydropyranyl, morphilino, 1,3-diazapane, 1,4-diazapane, 1,4-oxazepane, and 1,4-oxathiapane. A heterocycloalkyl group typically is a C₂-C₁₂ heterocycloalkyl group. The group may be a terminal group or a bridging group.

"Heterocycloalkylalkyl" refers to a heterocycloalkyl-alkyl- group in which the heterocycloalkyl and alkyl moieties are as defined herein. Exemplary heterocycloalkylalkyl groups include (2-tetrahydrofuryl)methyl, (2-tetrahydrothiofuranyl) methyl. The group may be a terminal group or a bridging group. If the group is a terminal group it is bonded to the remainder of the molecule through the alkyl group.

"Heterocycloalkylheteroalkyl" means a heterocycloalkyl-heteroalkyl- group in which the heterocycloalkyl and heteroalkyl moieties are as defined herein. The group may be a terminal group or a bridging group. If the group is a terminal group it is bonded to the remainder of the molecule through the heteroalkyl group.

"Heterocycloalkenyl" refers to a heterocycloalkyl group as defined herein but containing at least one double bond. A heterocycloalkenyl group typically is a C₂-C₁₂ heterocycloalkenyl group. The group may be a terminal group or a bridging group.

The term "therapeutically effective amount" or "effective amount" is an amount sufficient to effect beneficial or desired clinical results. An effective amount can be administered in one or more administrations. An effective amount is typically sufficient to palliate, ameliorate, stabilize, reverse, slow or delay the progression of the disease state. An effective amount for radioimaging is typically sufficient to identify the radionuclide in the subject.

The term "molecular recognition moiety" refers to an entity capable of binding to a particular molecular entity, typically a receptor location in the physiological environment. The term includes antibodies, proteins, peptides, carbohydrates, nucleic acids, oligonucleotides, oligosaccharides and liposomes.

The methods of synthesis of the present invent involve a transamination reaction in which a tertiary nitrogen atom at one end of the ligand is selectively displaced as described herein by reaction with a primary amine. The reaction may be carried out in any suitable solvent which is inert to the two reactants with the identity of the solvent being determined by the relative solubilities of the starting material ligand and the primary amine Examples of solvents that may be used include aliphatic, aromatic, or halogenated hydrocarbons such as benzene, toluene, xylenes; chlorobenzene, chloroform, methylene chloride, ethylene chloride; ethers and ethereal compounds such as dialkyl ether, ethylene glycol mono or-dialkyl ether, THF, dioxane; nitriles such as acetonitrile or 2-methoxypropionitrile; N,N-dialkylated amides such as dimethylformamide; and dimethyl acetamide, dimethylsulphoxide, tetramethylurea; as well as mixtures of these solvents with each other.

The reaction may be carried out at any of a number of suitable temperatures with the reaction temperature being able to be readily determined on a case by case basis and, in some instances, by the nature of the leaving group. Specifically where the leaving amine has a low boiling point such as diethyl amine (55°C) methyl ethyl amine (36°C) or dimethyl amine (7°C) it is highly desirable to carry out the reaction at a temperature in excess of the boiling point so that the amine is removed from the reaction mixture thus facilitating the reaction. Nevertheless the reaction temperature is typically carried out at from 0 to 100°C, more typically 50 to 80°C in refluxing solvent. In some embodiments the reaction is carried out at a temperature and pressure at which the leaving group (NHR⁵R⁶) is a gas to facilitate removal of the leaving group from the reaction mixture. The reaction mixture may be monitored by methods known in the art and the length of the reaction time will be based on a number of factors such as the temperature of the reaction and the identity of the reacting species. Nevertheless the reaction is typically conducted for from 1 to 24 hours.

Once the reaction has been completed the product is typically isolated using techniques known in the art. The isolated material may already contain a molecular recognition moiety or it may be such that it can readily be reacted with a suitable molecular recognition moiety. Examples of groups that can readily be reacted with a molecular recognition moiety include carboxylate groups (CO₂H) or sulfonate groups (SO₃H). These groups can readily be elaborated such that a molecular recognition moiety is attached using standard peptide coupling techniques.

In principle any of a wide range of biologically active molecular recognition units may be employed in the present invention with the only limitation being that the molecular recognition moiety used must contain a suitable terminal moiety for coupling to the end of the terminal residue of the compound made by the method of the invention as discussed above. For example where the terminal residue is a carboxylate or sulfonate residue the molecular recognition moiety most suitably has a terminal amine available for reaction. The coupling reactions of moieties of this type may be carried out in ways well known in the art and typically employ peptide synthesis techniques well known in the art which may involve either solid phase or liquid phase peptide synthesis techniques to be used.

Examples of compounds that may be produced using the methodology described above include:

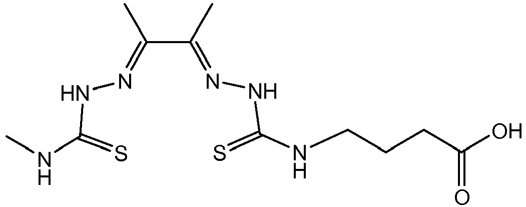

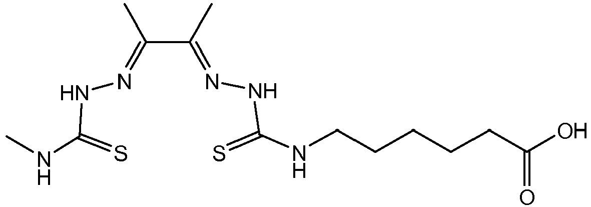

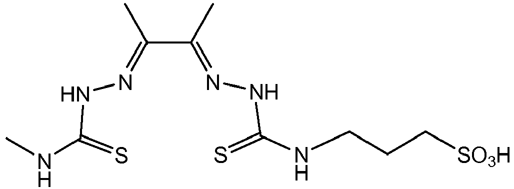

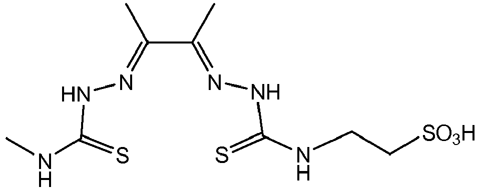

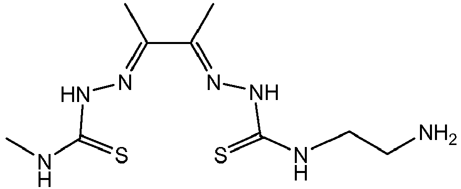

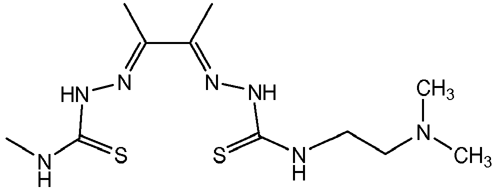

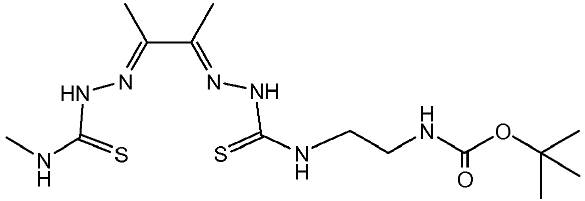

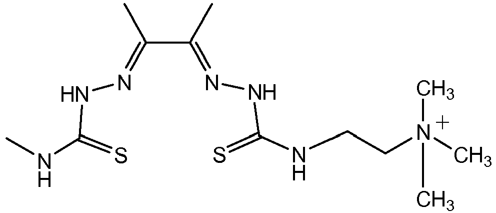

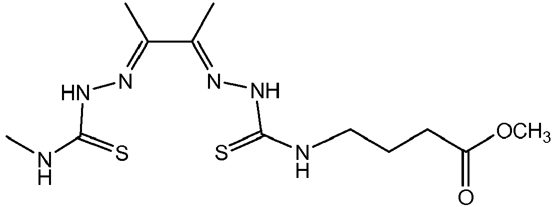

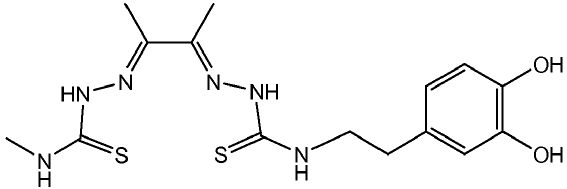

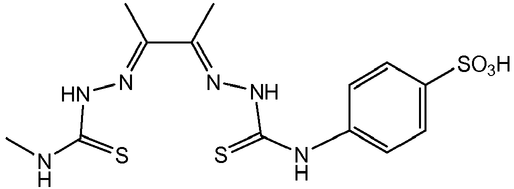

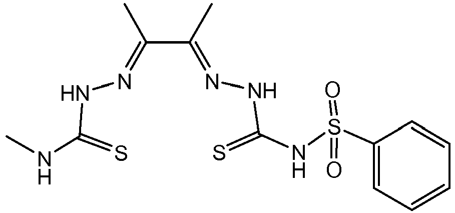

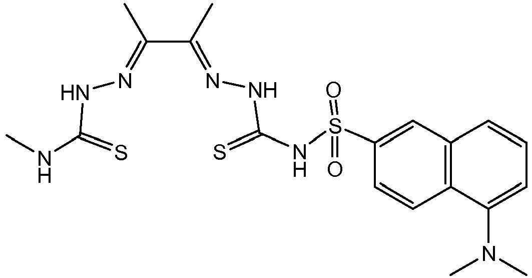

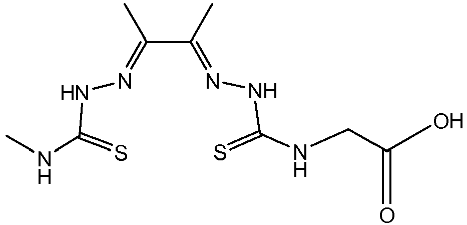

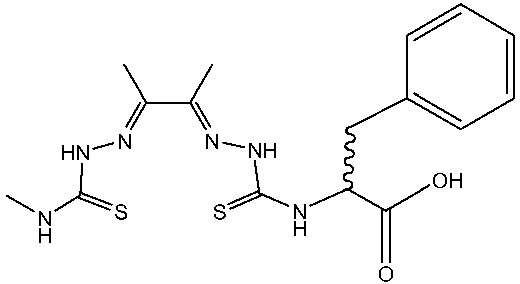

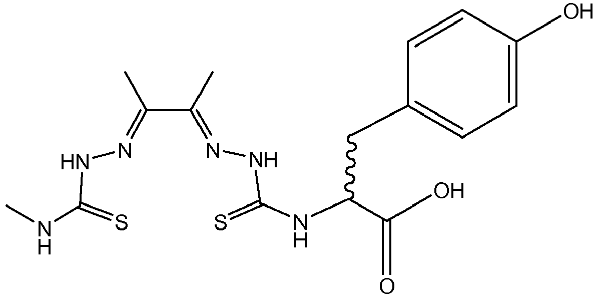

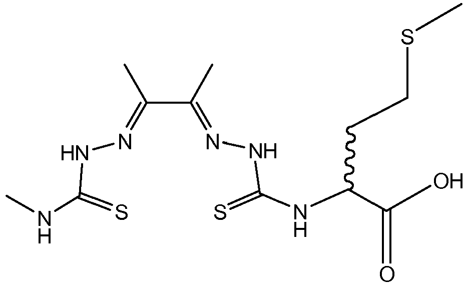

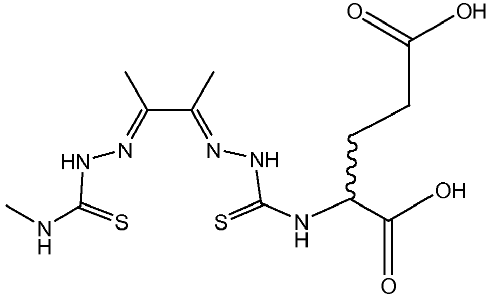

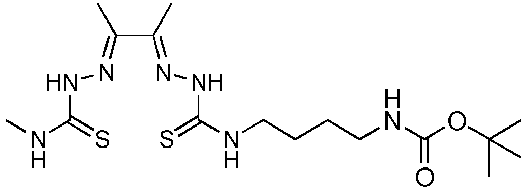

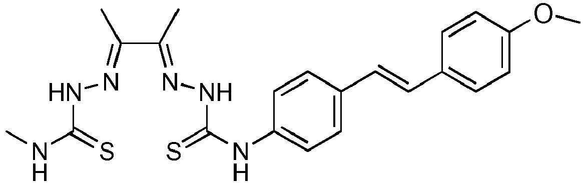

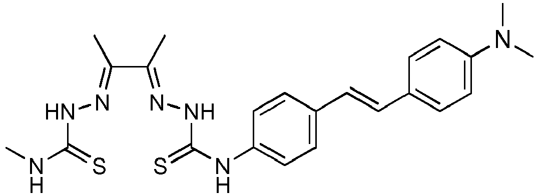

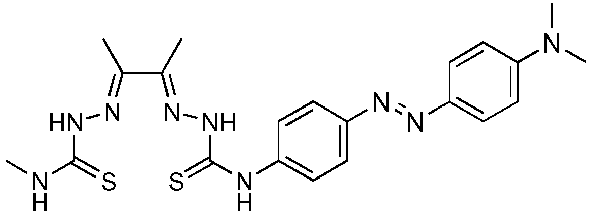

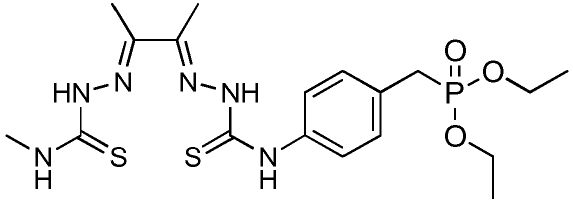

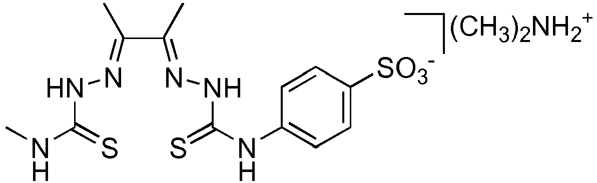

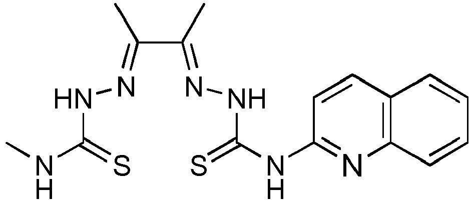

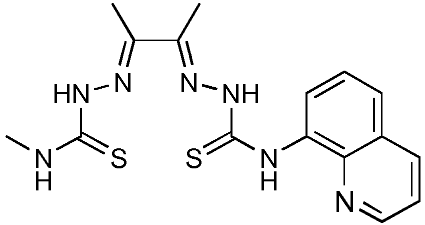

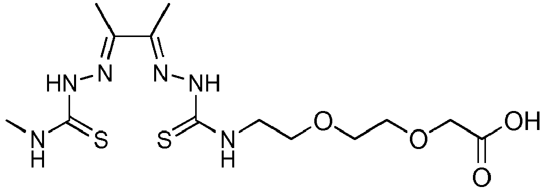

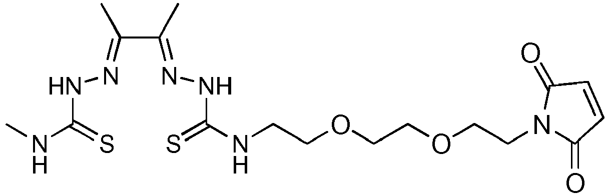

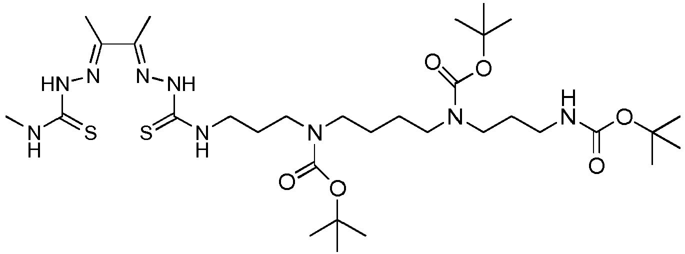

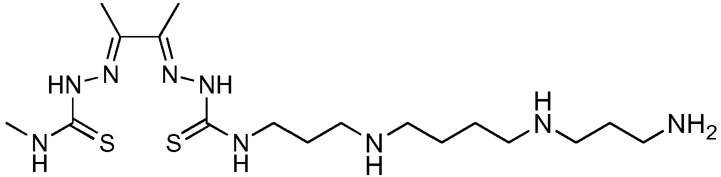

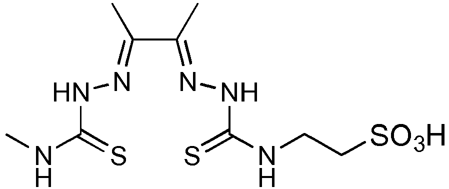

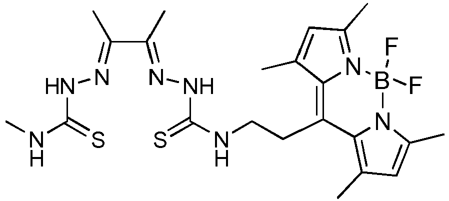

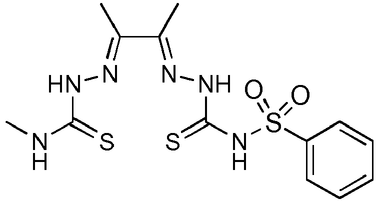

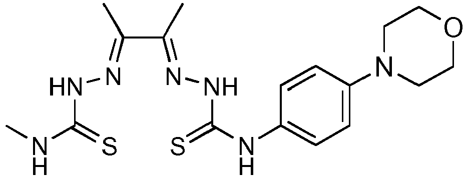

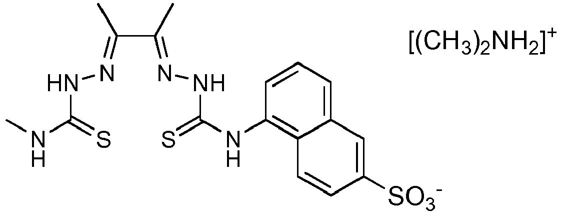

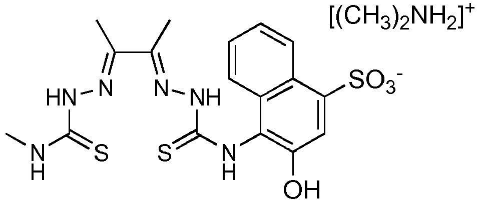

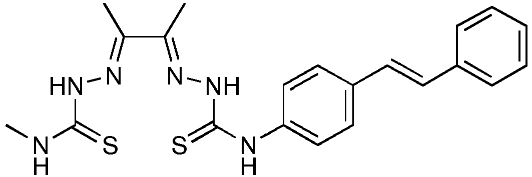

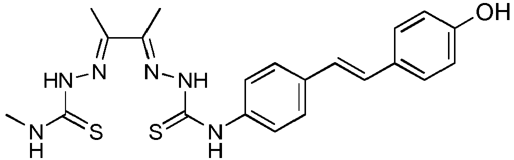

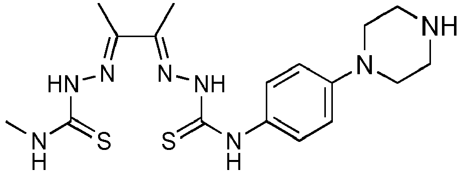

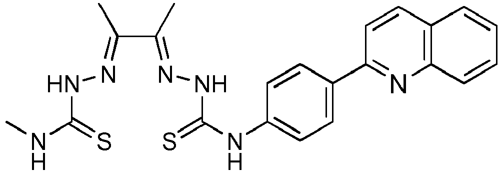

Examples of compounds containing a molecular recognition moiety include:

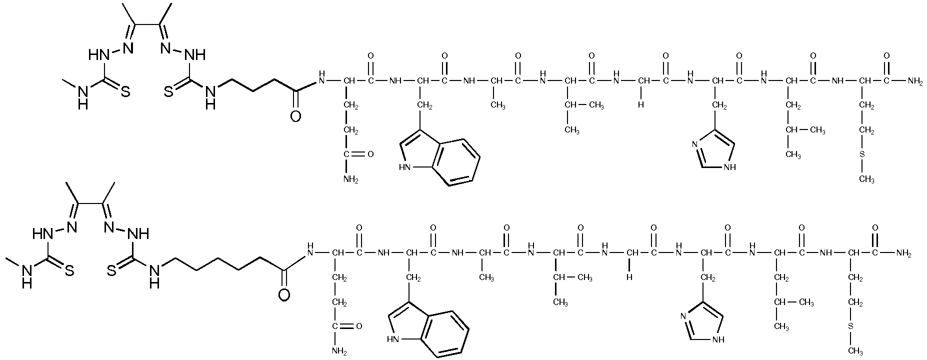

As discussed above the compounds made by the method of the invention are useful as they either have, or can be modified to contain a molecular recognition moiety.

The compounds made by the method of the invention are of particular use as they may be used to deliver metal to a subject and, where the compound contains a molecular recognition moiety, deliver the metal to a desired location in the body. In some embodiments of the complex made by the method of the invention described above the metal is selected from the group consisting of Iron, Nickel, Palladium, Cadmium, Manganese, Cobalt, Copper and Zinc. In some embodiments the metal is Copper or Zinc. In some specific embodiments the metal is Copper. In some specific embodiments the metal is Zinc.

The compounds made by the method of the invention containing a radionuclide complexed with the ligand may be used in either radiotherapy or in diagnostic imaging applications. In each instance both therapy and diagnostic imaging will rely on the molecular recognition moiety being involved in facilitating the localisation of the complex containing the radionuclide in the desired tissues or organs of the subject being treated/imaged.

In addition the compounds may be used in methods of treatment in which metal delivery to the subject is of interest. These metal treatments may be in the area of radiotherapy in which the metal is a radionuclide or they may be in other areas where non-radioactive metal delivery is beneficial.

Accordingly, the complexes made by the method of the invention may be used in the treatment or prophylaxis of a number of conditions in which metal delivery can prevent, alleviate or ameliorate the condition.

There are a number of conditions of this type. An example of conditions of this type is conditions associated with or caused by oxidative stress. It is known that many of the protective biological anti-oxidant mechanisms involve metal catalysed enzymes and thus metal delivery can serve to stimulate or re-start the activity of the biological anti-oxidant mechanisms leading to an overall anti-oxidant effect being achieved. The condition associated with or caused by oxidative stress may be selected from the group consisting of cardiovascular conditions, cancers, cataracts, neurological disorders such as Alzheimer's disease, prion diseases - including Creutzfeldt-Jakob Disease (CJD), and heart diseases, amyloidogenic amylotrophic lateral sclerosis (ALS), prion transmissible spongioform encephalopathies (TSE), cataracts, mitochondrial disorders, Menke's disease, Parkinson's disease and Huntington's disease.

The disorder may be a neuromuscular disorder selected from the group consisting of amyotrophic lateral sclerosis (ALS), mitochondrial/metabolic disease and Friedreich's ataxia.

The condition may be a neurological condition or a neurodegenerative disorder.

The term "neurological condition" is used herein in its broadest sense and refers to conditions in which various cell types of the nervous system are degenerated and/or have been damaged as a result of neurodegenerative disorders or injuries or exposures. In particular, complexes of formula (I) can be used for the treatment of resulting conditions, in which damage to cells of the nervous system has occurred due to surgical interventions, infections, exposure to toxic agents, tumours, nutritional deficits or metabolic disorders. In addition, the complex of formula (I) can be used for the treatment of the sequelae of neurodegenerative disorders, such as Alzheimer's disease, Parkinson's disease, multiple sclerosis, amylotrophic lateral sclerosis, epilepsy, drug abuse or drug addiction (alcohol, cocaine, heroin, amphetamine or the like), spinal cord disorders, dystrophy or degeneration of the neural retina (retinopathies) and peripheral neuropathies, such as diabetic neuropathy and/or the peripheral neuropathies induced by toxins. In relation to neurological conditions it is anticipated that the compounds made by the method of the invention may have an effect on neurons as well as other cells of the nervous system such as astrocytes, oligodendrocytes, schwann cells and the like

The term "neurodegenerative disorder" as used herein refers to an abnormality in which neuronal integrity is threatened. Neuronal integrity can be threatened when neuronal cells display decreased survival or when the neurons can no longer propagate a signal.

Neurological conditions that would be expected to be able to be treated with the complexes made by the method of the present invention include acute intermittent porphyria; adriamycin-induced cardiomyopathy; AIDS dementia and HIV-1 induced neurotoxicity; AD; ALS; atherosclerosis; cataract; cerebral ischaemia; cerebral palsy; cerebral tumour; chemotherapy-induced organ damage; cisplatin-induced nephrotoxicity; coronary artery bypass surgery; CJD and its new variant associated with "mad cow" disease; diabetic neuropathy; Down Syndrome; drowning; epilepsy and post-traumatic epilepsy; Friedrich's ataxia; frontotemporal dementia; glaucoma; glomerulopathy; haemochromatosis; haemodialysis; haemolysis; haemolytic uraemic syndrome (Weil's disease); Menke's disease; haemorrhagic stroke; Hallerboden-Spatz disease; heart attack and reperfusion injury; HD; Lewy body disease; intermittent claudication; ischaemic stroke; inflammatory bowel disease; macular degeneration; malaria; methanol-induced toxicity; meningitis (aseptic and tuberculous); motor neuron disease; multiple sclerosis; multiple system atrophy; myocardial ischaemia; neoplasia; Parkinson's disease; peri-natal asphyxia; Pick's disease; progressive supra-nuclear palsy; radiotherapy-induced organ damage; restenosis after angioplasty; retinopathy; senile dementia; schizophrenia; sepsis; septic shock; spongiform encephalopathies; subharrachnoid haemorrhage/cerebral vasospasm; subdural haematoma; surgical trauma, including neurosurgery; thalassemia; transient ischaemic attack (TIA); transplantation; vascular dementia; viral meningitis; and viral encephalitis.

Additionally, the complexes made by the method of the present invention may also be used to potentiate the effects of other treatments, for example to potentiate the neuroprotective effects of brain derived nerve growth factor.

The complexes made by the method of the invention may also be used to treat Anemia, Neutropenia, Copper deficiency Myelopathy, Copper deficiency Syndrome and Hyperzincaemia.

The complexes made by the method of the invention may also be used to treat conditions which induce oxidative damage of the central nervous system, including acute and chronic neurological disorders such as, cerebral ischaemia, stroke (ischaemic and haemorrhagic), subharrachnoid haemorrhage/cerebral vasospasm, cerebral tumour, AD, CJD and its new variant associated with "mad cow" disease, HD, PD, Friedrich's ataxia, cataract, dementia with Lewy body formation, multiple system atrophy, Hallerboden-Spatz disease, diffuse Lewy body disease, amylotrophic lateral sclerosis, motor neuron disease, multiple sclerosis, fatal familial insomnia, Gertsmann Straussler Sheinker disease and hereditary cerebral haemorrhage with amyoidoisis-Dutch type.

More particularly, the complexes made by the method of the invention may also be used to treat neurodegenerative amyloidosis. The neurodegenerative amyloidosis may be any condition in which neurological damage results from the deposition of amyloid. The amyloid may be formed from a variety of protein or polypeptide precursors, including but not limited to Aβ, synuclein, huntingtin, or prion protein.

Thus the condition may be selected from the group consisting of sporadic or familial AD, ALS, motor neuron disease, cataract, PD, Creutzfeldt-Jacob disease and its new variant associated with "mad cow" disease, HD, dementia with Lewy body formation, multiple system atrophy, Hallerboden-Spatz disease, and diffuse Lewy body disease.

The neurodegenerative amyloidosis may be an Aβ-related condition, such as AD or dementia associated with Down Syndrome or one of several forms of autosomal dominant forms of familial AD (reviewed in St George-Hyslop, 2000). Most preferably the Aβ-related condition is AD.

Prior to treatment the subject may have moderately or severely impaired cognitive function, as assessed by the AD Assessment Scale (ADAS)-cog test, for example an ADAS-cog value of 25 or greater.

In addition to slowing or arresting the cognitive decline of a subject, the complex made by the method of the invention may also be suitable for use in the treatment or prevention of neurodegenerative conditions, or may be suitable for use in alleviating the symptoms of neurodegenerative conditions. If administered to a subject who has been identified as having an increased risk of a predisposition to neurodegenerative conditions, or to a subject exhibiting pre-clinical manifestations of cognitive decline, such as Mild Cognitive Impairment or minimal progressive cognitive impairment, these methods and compounds may be able to prevent or delay the onset of clinical symptoms, in addition to the effect of slowing or reducing the rate of cognitive decline.

Currently AD and other dementias are usually not diagnosed until one or more warning symptoms have appeared. These symptoms constitute a syndrome known as Mild Cognitive Impairment (MCI), which was recently defined by the American Academy of Neurology, and refers to the clinical state of individuals who have memory impairment, but who are otherwise functioning well, and who do not meet clinical criteria for dementia (Petersen et al., 2001). Symptoms of MCI include:
(1) Memory loss which affects job skills
(2) Difficulty performing familiar tasks
(3) Problems with language
(4) Disorientation as to time and place (getting lost)
(5) Poor or decreased judgement
(6) Problems with abstract thinking
(7) Misplacing things
(8) Changes in mood or behaviour
(9) Changes in personality
(10) Loss of initiative.

MCI can be detected using conventional cognitive screening tests, such as the Mini Mental Status Exam, and the Memory Impairment Screen, and neuropsychological screening batteries.

Another condition that may be able to be treated by metal delivery is cancer. The term "cancer" describes any array of different diseases linked by cumulative multiple genetic mutations, which result in the activation of oncogenes and/or the inactivation of tumour suppressor genes and/or linked by uncontrolled cellular proliferation. The cause and source of these mutations differs between different cancers of human body organs.

Cancer may be brain cancer, which includes a brain tumour. A brain cancer or tumour may be a glioma or non-glioma brain tumour. The term "cancer" and "tumour" may be used interchangeably herein. "Cancer" may include any one of the following states: glioma, adenoma, blastoma, carcinoma, sarcoma and inclusive of any one of Medulloblastoma, Ependymoma, Astrocytoma, Optical nerve glioma, Brain stem glioma, Oligodendroglioma, Gangliogliomas, Craniopharyngioma or Pineal Region Tumours. Reference to a "glioma" includes GMB, astrocytoma and anaplastic astrocytoma or related brain cancers.

The complexes made by the method of the present invention may also be able to be used to treat tau related disorders. Tau protein is an important protein as it is the protein expressed in the central nervous system and plays a critical role in the neuronal architecture by stabilizing intracellular microtubule network. Accordingly any impairment of the physiological role of the tau protein either by truncation, hyper-phosphorylation or by disturbing the balance between the six naturally occurring tau isoforms is detrimental to the subject and leads to the formation of neurofibrillary tangles (NFT), dystrophic neurites and neuropil threads. The major protein subunit of these structures is microtubule associated protein tau. The amount of NFT found in autopsies of AD patients correlates with clinical symptoms including intellectual decline. Accordingly tau protein plays a critical role in AD pathology. The recent discovery of cosegregation of specific mutations in the tau gene with the disease frontotemporal dementia with Parkinsonism linked to chromosome 17 (FTDP-17) has confirmed that certain abnormalities in the tau protein can be a primary cause of neurodegeneration and dementia in affected individuals.

Without wishing to be bound by theory it is felt that the activity of the complexes made by the method of the present invention to reduce levels of tau phosphorylation is as a result of their ability to deliver metal to cells and hence their anti-oxidant activity. It is felt that the ability of the complexes to act as anti-oxidants mean that they provide protection from OS which is desirable as OS can lead to hyper-phosphorylation of tau and cell dysfunction. As a consequence the ability of these complexes to deliver biologically important metals to cells allows them to function as anti-oxidants (especially where the oxidative stress is caused by metal deficiency) which in turn means the metal complexes may have the ability to prevent (or treat) tau-opathies.

There are a number of disorders or conditions that are recognized as being tau disorders or more colloquially Tauopathies. Disorders of this type include Richardson's syndrome, Progressive Supranuclear Palsy, Agryrophilic grain disease, corticobasal degeneration, Pick's disease, frontotemporal dementia linked with parkinsonism linked to chromosome 17 9FTDP-17), post-encephalitic parkinsonism (PEP), dementia pugilistica, Down Syndrome, Alzheimer's disease, Familial British dementia, Familial Danish dementia, Parkinson's disease, Parkinson's Disease complex of Guam (PDC), myotonic dystrophy, Hallevorden-Spatz disease, and Niemann-Pick type C.

The complexes may also be used in the treatment of an Abeta related disorder. A number of Abeta disorders are known including disorders selected from the group consisting of Parkinson's disease, Alzheimer's disease, Multiple sclerosis, Neuropathies, Huntington's disease, Prion disease, motor neurone disease, Amyotrophic lateral sclerosis (ALS), Menke's disease, and amyloidoses.

As the complexes made by the method of the invention have also been shown to be able to deliver metal to cells they have the ability to influence matrix metallo-proteinases (MMP's). Matrix metalloproteinases (MMPs) are a family of zinc-and calcium-dependent secreted or membrane anchored endopeptidases which play a number of important biological functions. MMPs are involved in many physiological processes but also take part in the pathophysiological mechanisms responsible for a wide range of diseases. Pathological expression and activation of MMPs are associated with cancer, atherosclerosis, stroke, arthritis, periodontal disease, multiple sclerosis and liver fibrosis. Accordingly the complexes made by the method of the invention have the potential to influence these conditions.

In general depending upon the condition to be treated an effective amount of the metal complex (containing the desired metal of choice) will be administered to the patient or subject. The metal chosen will depend upon the condition and the state of the patient.

In relation to the use of the radiolabelled compounds of formula (III) it is anticipated that these will be used by administration of an effective amount of the radiolabelled compound to a subject followed by monitoring of the subject after a suitable time period to determine if the radiolabelled compound has localised at a particular location in the body or whether the compound is broadly speaking evenly distributed through the body. As a general rule where the radio labelled compound is localised in tissue or an organ of the body this is indicative of the presence in that tissue or organ of a moiety that is recognised by the particular molecular recognition moiety used.

Accordingly judicious selection of a molecular recognition moiety is important in determining the efficacy of any of the radiolabelled compounds made by the method of the invention in diagnostic imaging applications. In this regard a wide range of molecular recognition moieties are known in the art which are well characterised and which are known to selectively target certain receptors in the body. In particular a number of molecular recognition moieties are known that target tissue or organs when the patient is suffering from certain medical conditions. Examples of molecular recognition moieties that are known and may be use in this invention include Octreotate, octreotide, [Tyr³]-octreotate, [Tyr¹]-octreotate, bombesin, bombesin(7-14), gastrin releasing peptide, single amino acids, penetratin, annexin V, TAT, cyclic RGD, glucose, glucosamine (and extented carbohydrates), folic acid, neurotensin, neuropeptide Y, cholecystokinin (CCK) analogues, vasoactive intestinal peptide (VIP), substance P, alpha-melanocyte-stimulating hormone (MSH). For example, certain cancers are known to over express somatostatin receptors and so the molecular recognition moiety may be one which targets these receptors. An example of a molecular recognition moiety of this type is [Tyr³]-octreotate. In other examples the molecular recognition moiety is bombesin which is known to target breast and pancreatic cancers.

The monitoring of the subject for the location of the radiolabelled material will typically provide the analyst with information regarding the location of the radiolabelled material and hence the location of any material that is targeted by the molecular recognition moiety (such as cancerous tissue). An effective amount of the compounds made by the method of the invention will depend upon a number of factors and will by necessity involve a balance between the amount of radioactivity required to achieve the desired radio imaging effect and the general interest in not exposing the subject (or their tissues or organs) to any unnecessary levels of radiation which may be harmful.

The methods of treatment which are directed towards radiotherapy involve administration of a compound of formula (III) complexed to a radionuclide. The compounds of formula (III) typically contain a molecular recognition moiety in order to deliver the radionuclide to the desired location in the body where its mode of action is desired. As discussed above examples of such molecular recognition moieties are known in the art and a skilled artisan can select the appropriate molecular recognition moiety to target the desired tissue in the body to be treated.

A therapeutically effective amount can be readily determined by an attending clinician by the use of conventional techniques and by observing results obtained under analogous circumstances. In determining the therapeutically effective amount a number of factors are to be considered including but not limited to, the species of animal, its size, age and general health, the specific condition involved, the severity of the condition, the response of the patient to treatment, the particular radio labelled compound administered, the mode of administration, the bioavailability of the preparation administered, the dose regime selected, the use of other medications and other relevant circumstances.

In addition the treatment regime will typically involve a number of cycles of radiation treatment with the cycles being continued until such time as the condition has been ameliorated. Once again the optimal number of cycles and the spacing between each treatment cycle will depend upon a number of factors such as the severity of the condition being treated, the health (or lack thereof) of the subject being treated and their reaction to radiotherapy. In general the optimal dosage amount and the optimal treatment regime can be readily determined by a skilled addressee in the art using well known techniques.

The compounds made by the method of the invention they can be administered in any form or mode which makes the compound available for the desired application (imaging or radio therapy). One skilled in the art of preparing formulations of this type can readily select the proper form and mode of administration depending upon the particular characteristics of the compound selected, the condition to be treated, the stage of the condition to be treated and other relevant circumstances. We refer the reader to Remingtons Pharmaceutical Sciences, 19th edition, Mack Publishing Co. (1995) for further information.

The compounds made by the method of the present invention can be administered alone or in the form of a pharmaceutical composition in combination with a pharmaceutically acceptable carrier, diluent or excipient. The compounds made by the method of the invention, while effective themselves, are typically formulated and administered in the form of their pharmaceutically acceptable salts as these forms are typically more stable, more easily crystallised and have increased solubility.

The compounds are, however, typically used in the form of pharmaceutical compositions which are formulated depending on the desired mode of administration. The compositions are prepared in manners well known in the art.

A pharmaceutical pack or kit comprising one or more containers filled with one or more of the ingredients of the pharmaceutical compositions made by the method of the invention may be provided. In such a pack or kit can be found at least one container having a unit dosage of the agent(s). Conveniently, in the kits, single dosages can be provided in sterile vials so that the clinician can employ the vials directly, where the vials will have the desired amount and concentration of compound and radio nucleotide which may be admixed prior to use. Associated with such container(s) can be various written materials such as instructions for use, or a notice in the form prescribed by a governmental agency regulating the manufacture, use or sale of pharmaceuticals, imaging agents or biological products, which notice reflects approval by the agency of manufacture, use or sale for human administration.

The compounds made by the method of the invention may be used or administered in combination with one or more additional drug(s) that are anti-cancer drugs and/or procedures (e.g. surgery, radiotherapy) for the treatment of the disorder/diseases mentioned. The components can be administered in the same formulation or in separate formulations. If administered in separate formulations the compounds made by the method of the invention may be administered sequentially or simultaneously with the other drug(s).

In addition to being able to be administered in combination with one or more additional drugs that include anti-cancer drugs, the compounds made by the method of the invention may be used in a combination therapy. When this is done the compounds are typically administered in combination with each other. Thus one or more of the compounds made by the method of the invention may be administered either simultaneously (as a combined preparation) or sequentially in order to achieve a desired effect. This is especially desirable where the therapeutic profile of each compound is different such that the combined effect of the two drugs provides an improved therapeutic result.

Pharmaceutical compositions made by the method of this invention for parenteral injection comprise pharmaceutically acceptable sterile aqueous or nonaqueous solutions, dispersions, suspensions or emulsions as well as sterile powders for reconstitution into sterile injectable solutions or dispersions just prior to use. Examples of suitable aqueous and nonaqueous carriers, diluents, solvents or vehicles include water, ethanol, polyols (such as glycerol, propylene glycol, polyethylene glycol, and the like), and suitable mixtures thereof, vegetable oils (such as olive oil), and injectable organic esters such as ethyl oleate. Proper fluidity can be maintained, for example, by the use of coating materials such as lecithin, by the maintenance of the required particle size in the case of dispersions, and by the use of surfactants.

These compositions may also contain adjuvants such as preservative, wetting agents, emulsifying agents, and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents such as sugars, sodium chloride, and the like. Prolonged absorption of the injectable pharmaceutical form may be brought about by the inclusion of agents that delay absorption such as aluminium monostearate and gelatin.

If desired, and for more effective distribution, the compounds can be incorporated into slow release or targeted delivery systems such as polymer matrices, liposomes, and microspheres.

The injectable formulations can be sterilized, for example, by filtration through a bacterial-retaining filter, or by incorporating sterilizing agents in the form of sterile solid compositions that can be dissolved or dispersed in sterile water or other sterile injectable medium just prior to use.

Solid dosage forms for oral administration include capsules, tablets, pills, powders, and granules. In such solid dosage forms, the active compound is mixed with at least one inert, pharmaceutically acceptable excipient or carrier such as sodium citrate or dicalcium phosphate and/or a) fillers or extenders such as starches, lactose, sucrose, glucose, mannitol, and silicic acid, b) binders such as, for example, carboxymethylcellulose, alginates, gelatin, polyvinylpyrrolidone, sucrose, and acacia, c) humectants such as glycerol, d) disintegrating agents such as agar-agar, calcium carbonate, potato or tapioca starch, alginic acid, certain silicates, and sodium carbonate, e) solution retarding agents such as paraffin, f) absorption accelerators such as quaternary ammonium compounds, g) wetting agents such as, for example, cetyl alcohol and glycerol monostearate, h) absorbents such as kaolin and bentonite clay, and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using such excipients as lactose or milk sugar as well as high molecular weight polyethylene glycols and the like.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract, optionally, in a delayed manner. Examples of embedding compositions which can be used include polymeric substances and waxes.

If desired, and for more effective distribution, the compounds can be incorporated into slow release or targeted delivery systems such as polymer matrices, liposomes, and microspheres.

The active compounds can also be in microencapsulated form, if appropriate, with one or more of the above-mentioned excipients.

Liquid dosage forms for oral administration include pharmaceutically acceptable emulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethyl formamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, and perfuming agents.

Suspensions, in addition to the active compounds, may contain suspending agents as, for example, ethoxylated isostearyl alcohols, polyoxyethylene sorbitol and sorbitan esters, microcrystalline cellulose, aluminium metahydroxide, bentonite, agar-agar, and tragacanth, and mixtures thereof.

As discussed above, the compounds of the embodiments may be useful for treating and/or detecting proliferative diseases. Examples of such cell proliferative diseases or conditions include cancer (include any metastases), psoriasis, and smooth muscle cell proliferative disorders such as restenosis. The compounds made by the method of the present invention may be particularly useful for treating and/or detecting tumours such as breast cancer, colon cancer, lung cancer, ovarian cancer, prostate cancer, head and/or neck cancer, or renal, gastric, pancreatic cancer and brain cancer as well as hematologic malignancies such as lymphoma and leukaemia. In addition, the compounds made by the method of the present invention may be useful for treating and/or detecting a proliferative disease that is refractory to the treatment and/or detecting with other anti-cancer drugs; and for treating and/or detecting hyperproliferative conditions such as leukaemia's, psoriasis and restenosis. In other embodiments, compounds made by the method of this invention can be used to treat and/or detect pre-cancer conditions or hyperplasia including familial adenomatous polyposis, colonic adenomatous polyps, myeloid dysplasia, endometrial dysplasia, endometrial hyperplasia with atypia, cervical dysplasia, vaginal intraepithelial neoplasia, benign prostatic hyperplasia, papillomas of the larynx, actinic and solar keratosis, seborrheic keratosis and keratoacanthoma.

### SYNTHESIS OF COMPOUNDS MADE BY THE METHOD OF THE INVENTION

The agents of the various embodiments may be prepared using the reaction routes and synthesis schemes as described below, employing the techniques available in the art using starting materials that are either readily available or that which can be synthesized from available starting materials. The preparation of particular compounds of the embodiments is described in detail in the following examples, but the artisan will recognize that the chemical reactions described may be readily adapted to prepare a number of other agents of the various embodiments. For example, the synthesis of non-exemplified compounds may be successfully performed by modifications apparent to those skilled in the art, e.g. by appropriately protecting interfering groups, by changing to other suitable reagents known in the art, or by making routine modifications of reaction conditions. A list of suitable protecting groups in organic synthesis can be found in T.W. Greene's Protective Groups in Organic Synthesis, 3rd Edition, John Wiley & Sons, 1991. Alternatively, other reactions disclosed herein or known in the art will be recognized as having applicability for preparing other compounds of the various embodiments.

Reagents useful for synthesizing compounds may be obtained or prepared according to techniques known in the art. The general reaction scheme is shown in scheme 1.

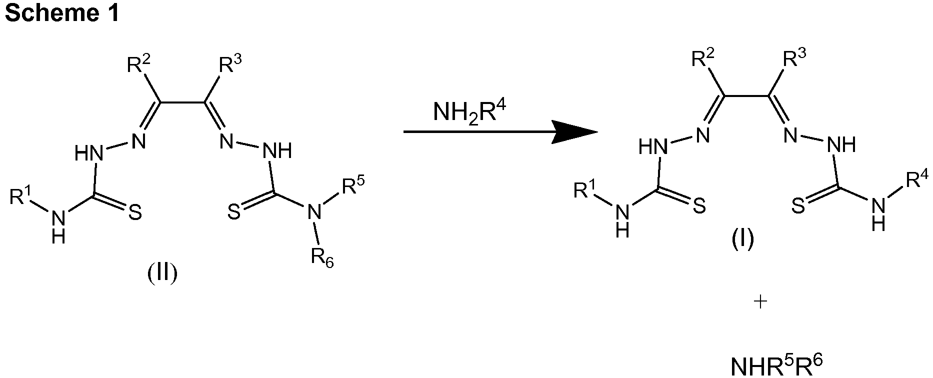

Accordingly a suitably substituted bisthiosemicarbazone of formula (II) is reacted with an amine of formula NH₂R⁴ in a displacement reaction leading to formation of the compound of formula (I). The compounds thus produced may be easily converted into their copper salts by reaction with copper acetate in a suitable solvent.

### EXAMPLES

In the examples described below, unless otherwise indicated, all temperatures in the following description are in degrees Celsius and all parts and percentages are by weight, unless indicated otherwise.

Various starting materials and other reagents were purchased from commercial suppliers, such as Aldrich Chemical Company or Lancaster Synthesis Ltd., and used without further purification, unless otherwise indicated. Tetrahydrofuran (THF) and N,N-dimethylformamide (DMF) were purchased from Aldrich in SureSeal bottles and used as received. All solvents were purified by using standard methods in the art, unless otherwise indicated.

The reactions set forth below were performed under a positive pressure of nitrogen, argon or with a drying tube, at ambient temperature (unless otherwise stated), in anhydrous solvents, and the reaction flasks are fitted with rubber septa for the introduction of substrates and reagents via syringe. Glassware was oven-dried and/or heat-dried.

Work-ups were typically done by doubling the reaction volume with the reaction solvent or extraction solvent and then washing with the indicated aqueous solutions using 25% by volume of the extraction volume (unless otherwise indicated). Product solutions were dried over anhydrous sodium sulfate prior to filtration, and evaporation of the solvents was under reduced pressure on a rotary evaporator and noted as solvents removed in vacuo.

Mass spectra were recorded in the positive ion mode on an Agilent 6510 Q-TOF LC/MS Mass Spectrometer coupled to an Agilent 1100 LC system (Agilent, Palo Alto, CA). Data were acquired and reference mass corrected via a dual-spray electrospray ionisation source, using the factory-defined calibration procedure. Each scan or data point on the Total Ion Chromatogram is an average of 9652 transients, producing 1.02 scans s⁻¹. Spectra were created by averaging the scans across each peak. Mass spectrometer conditions: fragmentor: 200 - 300 V; drying gas flow: 7 L/min; nebuliser: 30 psi; drying gas temp: 325°C; V_{cap}: 4000 V; skimmer: 65 V; OCT R_{f}V: 750 V; scan range acquired: 150 - 3000 *m*/*z.*

HPLC-MS traces were recorded using an Agilent Eclipse Plus C18 column (5 µm, 2.1 x 150 mm) coupled to the Agilent 6510 Q-TOF LC/MS Mass Spectrometer described above. 1 µL aliquots of each sample were injected onto the column using the Agilent 1100 LC system, with a flow rate of 0.5 mL/min. Data acquisition parameters are the same as those described above for mass spectra, with the exception of the fragmentor (fragmentor voltage: 100 V).

NMR spectra were recorded on a Varian FT-NMR 500 spectrometer operating at 500 MHz for ¹H NMR and 125.7 MHz for ¹³C-NMR. NMR spectra are obtained as d₆-DMSO solutions (reported in ppm), using residual solvent as the reference standard (2.50 ppm respectively). Other NMR solvents were used as needed. When peak multiplicities are reported, the following abbreviations are used: s = singlet, d = doublet, t = triplet, m = multiplet, br = broadened, dd = doublet of doublets, dt = doublet of triplets. Coupling constants, when given, are reported in Hertz.

Semi-preparative HPLC purifications were performed using an Agilent 1200 Series HPLC system with a 5 mL/min flow rate. Solvent gradients and column specifications are described in the examples. An automated Agilent 1200 fraction collector collected 1 - 3 mL fractions and fraction collection was based on UV-Vis detection at 214 or 220 nm, with a lower threshold limit between 100 - 400 mAU. Each fraction was analysed using MS and analytical HPLC.

Analytical HPLC traces were acquired using an Agilent 1200 Series HPLC system and an Agilent Zorbax Eclipse XDB-C18 column (4.6 x 150 mm, 5 µm) with a 1 mL/ min flow rate and UV spectroscopic detection at 214 nm, 220 nm and 270 nm.

UV-Vis spectra were acquired on a Cary 300 Bio UV-Vis spectrophotometer, from 800 - 200 nm at 0.500 nm data intervals with a 300.00 nm/min scan rate.

Voltametric experiments were performed with an Autolab (Eco Chemie, Utrecht, Netherlands) computer-controlled electrochemical workstation. A standard three-electrode arrangement was used with a glassy carbon disk (d, 3 mm) as working electrode, a Pt wire as auxiliary electrode and a Ag/AgCl reference electrode (silver wire in H₂O (KCI (0.1 M) AgNO₃ (0.01 M)). Scan rate: 100 mV/s, sample interval: 1.06 mV, sensitivity: 1 x 10⁻⁴ A.

HPLC traces of radiolabelled peptides were acquired using a Waters Comosil C18 column (4.6 x 150 mm) coupled to a Shimadzu LC-20AT with a sodium iodide scintillation detector and a UV-Vis detector. 100 µL aliquots of each radiolabelled sample were injected onto the column, using a flow rate of 1 mL/min.

The following examples are intended to illustrate the embodiments disclosed and are not to be construed as being limitations thereto. Additional compounds, other than those described below, may be prepared using the above described reaction scheme or appropriate variations or modifications thereof.

### Reference Example 1 Synthesis of an exemplary starting material monoketo-thiosemicarbazone,

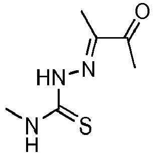

A solution of 2,3-butanedione (2.39 g, 27.7 mmol) in distilled water (50 mL) was acidified with a few drops of conc. HCl (36 %) and cooled to 5°C. 4-methyl-3-thiosemicarbazide (2.65 g, 25.2 mmol) was added in small portions to the stirred cold solution over 1.5 h to produce a white precipitate, which was further stirred for 40 min. The precipitate was extracted into chloroform (50 + 50 + 40 mL) and the extracts were combined, dried over MgSO₄.3H₂O, filtered and concentrated. N-pentane was added to the solution until slight turbidity and then it was cooled to -20°C to give white needles. The product was collected by filtration, washed with n-pentane and dried to give 1 (3.26 g, 18.82 mmol, 75 %). ¹H NMR (d₆-DMSO, 500 MHz): δ 1.96, 3H, s, CH₃; δ 2.42, 3H, s, CH₃; δ 3.05, 3H, d, ³J_{HH} = 4.5 Hz, NH-C*H*₃; δ 8.61, 1 H, m, NH; δ 10.61, 1H, s, NH.

### Reference Example 2 Synthesis of an exemplary compound of formula (II) Diacetyl-bis-(N'⁴-dimethyl, N"⁴-methylthiosemicarbazone) (L¹H₂)

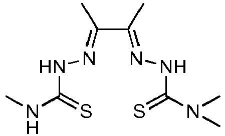

To a solution of the compound of example 1 (0.92 g, 5.3 mmol) in DMF (3 mL) was added 4,4-dimethyl-3-thiosemicarbazide (0.76 g, 6.4 mmol, 1.2 eq.) and acetic acid (5 drops, glacial). The resulting solution was stirred at room temperature for 48 h. A yellow solid precipitated from solution upon addition of water (50 mL). The suspension was cooled in an ice bath before the bright yellow solid was collected by filtration, washed with water (x1), ethanol (x2) and diethyl ether (x3) and dried to give the titled compound. (1.30 g, 4.7 mmol, 89 %). ¹H NMR (d₆-DMSO, 500 MHz): δ2.15,

### Example 1 - Synthesis of starting material monoketo-thiosemicarbazone

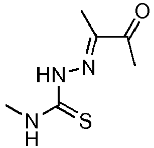

A solution of 2,3-butanedione (2.39 g, 27.7 mmol) in distilled water (50 mL) was acidified with a few drops of conc. HCl (36 %) and cooled to 5°C. 4-methyl-3-thiosemicarbazide (2.65 g, 25.2 mmol) was added in small portions to the stirred cold solution over 1.5 h to produce a white precipitate, which was further stirred for 40 min. The precipitate was extracted into chloroform (50 + 50 + 40 mL) and the extracts were combined, dried over MgSO₄.3H₂O, filtered and concentrated. N-pentane was added to the solution until slight turbidity and then it was cooled to -20°C to give white needles. The product was collected by filtration, washed with n-pentane and dried to give 1 (3.26 g, 18.82 mmol, 75 %). ¹H NMR (d₆-DMSO, 500 MHz): δ 1.96, 3H, s, CH₃; δ 2.42, 3H, s, CH₃; δ 3.05, 3H, d, ³J_{HH} = 4.5 Hz, NH-C*H*₃; δ 8.61, 1 H, m, NH; δ 10.61, 1H, s, NH.

### Example 2

### Diacetyl-bis-(N'⁴-dimethyl, N"⁴-methylthiosemicarbazone) (L¹H₂)

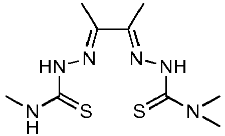

To a solution of the compound of example 1 (0.92 g, 5.3 mmol) in DMF (3 mL) was added 4,4-dimethyl-3-thiosemicarbazide (0.76 g, 6.4 mmol, 1.2 eq.) and acetic acid (5 drops, glacial). The resulting solution was stirred at room temperature for 48 h. A yellow solid precipitated from solution upon addition of water (50 mL). The suspension was cooled in an ice bath before the bright yellow solid was collected by filtration, washed with water (x1), ethanol (x2) and diethyl ether (x3) and dried to give the titled compound. (1.30 g, 4.7 mmol, 89 %). ¹H NMR (d₆-DMSO, 500 MHz): δ 2.15, 3H, s, CH₃; δ 2.19, 3H, s, CH₃; δ 3.03, 3H, d, ³J_{HH} = 4.5 Hz, NH-C*H*₃; δ 3.27, s, 6H, (CH₃)₂; δ 8.36, bq, 1 H, ³J_{HH} = 4.5 Hz, N*H*-CH₃; δ 9.49, bs, 1 H, NH; δ 10.16, bs, 1 H, NH. ¹³C NMR (125 MHz): δ 11.1, CH₃; δ 11.4, CH₃; δ 31.2, NH-CH₃; δ 42.3, (CH₃)₂; δ 148.0, C=N; δ 149.4, C=N; δ 178.5, C=S; δ 181.7, C=S. MS: (+ve ion) *m*/*z* [(L¹H₂) + H⁺]⁺ 275.3 (experimental), 275.1 (calculated), (-ve ion) *m*/*z* [(L¹H₂) - H⁺]⁻ 273.3 (experimental), 273.1 (calculated). Crystals suitable for single crystal X-ray crystallography were grown from a concentrated solution in DMSO.

### Example 3

### Diacetyl-bis-(N'⁴-butyric acid, N"⁴-methylthiosemicarbazone), (L²H₂)

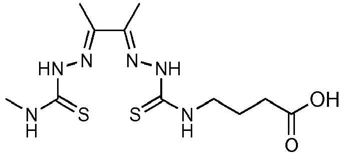

To a stirring suspension of the compound of example 2 (0.28 g, 1.0 mmol) in acetonitrile (30 mL) was added γ-aminobutyric acid (0.21 g, 2.0 mmol, 2 eq.). The resulting yellow suspension was heated at reflux for 34 h under an atmosphere of N₂. The resulting light cream suspension was cooled to room temperature and the white solid was collected by filtration, washed with HCl (3 %, 3 x 3.5 mL), acetonitrile (x1) and diethyl ether (x3) and dried to give the desired compound (**L**²H₂) (0.28 g, 0.8 mmol, 81 %). ¹H NMR (d₆-DMSO, 500 MHz): δ 1.81, 2H, m, CH₂-C*H*₂-CH₂; δ 2.21, 6H, s, CH₃; δ 2.25, 2H, t, ³J_{HH} = 7.5 Hz, C*H*₂-COOH; δ 3.02, 3H, d, ³J_{HH} = 4.5 Hz, NH-C*H*₃; δ 3.58, 2H, m, N*H*-CH₂-CH2; δ 8.38, 1H, q, ³J_{HH} = 4.5 Hz, N*H*-CH₃; δ 8.44, 1H, t, ³J_{HH} = 6 Hz, N*H*-CH₂; δ 10.18, 1H, s, NH; δ 10.23, 1H, s, NH. ¹³C NMR (125 MHz): δ 11.6, CH₃; δ 11.7, CH₃; δ 24.2, CH₂-CH₂-CH₂; δ 31.2, NH-CH₃, CH₂-COOH; δ 43.2, NH-CH₂; δ 147.9, C=N; δ 148.1, C=N; δ 174.2, C=O; δ177.8, C=S; δ 178.5, C=S. MS: (+ve ion) *m*/*z* [(L²H₂) + H⁺]⁺ 333.4 (experimental), 333.1 (calculated), (-ve ion) *m*/*z* [(L²H₂) - H⁺]- 331.3 (experimental), 331.1 (calculated).

### Reference Example 4

### Cu-diacetyl-bis-(N'⁴-butyric acid, N"⁴-methylthiosemicarbazone), Cu(L²)

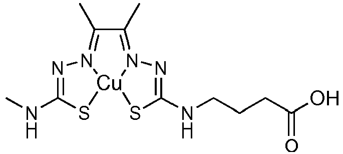

To a solution of the compound of example 3 (0.11 g, 0.3 mmol) in DMF (2 mL) stirring at room temperature was added Cu(OAc)₂.H₂O (0.07 g, 0.4 mmol). The red/brown solution was stirred at room temperature for 17 h. A brown solid was precipitated upon addition of water (40 mL) to the solution. The solid was collected by filtration, washed with water, ethanol and diethyl ether and dried to give **Cu(L²)** (0.09 g, 0.2 mmol, 70 %). MS: (+ve ion) **[Cu(L²)** + H⁺]⁺ *m*/*z* 100% 394.02994 (experimental), 394.03069 (calculated). RP HPLC: R_{T} = 9.879 min.

### Example 5

### Diacetyl-bis-(N'⁴-hexanoic acid, N"⁴-methylthiosemicarbazone), L³H₂

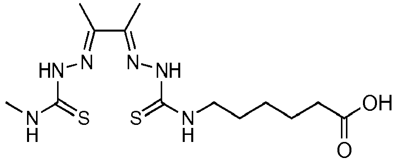

Following the same procedure employed in example 3, the product of example 2 (0.31 g, 1.1 mmol) and 6-aminocaproic acid (0.30 g, 2.3 mmol) were used to prepare the titled compound **L³**H₂ (0.33 g, 81 %). ¹H NMR (d₆ DMSO, 500 MHz): δ 1.29, 2H, m, NH-CH₂-C*H*₂-CH₂; δ 1.55, 4H, m, N*H*-CH₂-CH₂, C*H*₂-CH₂-COOH; δ2.20, 6H, s, CH₃; δ2.21, 2H, t, ³J_{HH} = 7.5 Hz, C*H*₂-COOH; δ 3.02, 3H, d, ³J_{HH} = 5 Hz, NH-C*H*₃; δ3.55, 2H, m, NH-C*H*₂; δ8.37, 2H, m, N*H*-CH₂, N*H*-CH₃; δ10.14, 1H, s, NH; δ 10.21, 1 H, s, NH; δ11.97, 1 H, bs, OH. ¹³C NMR (125 MHz): δ11.7, CH₃; δ 24.2, NH-CH₂-CH₂; δ 25.9, NH-CH₂-CH₂-CH₂; δ 28.4, CH₂-CH₂-COOH; δ 31.2, NH-CH₃; 9 33.6, CH₂-COOH; δ 43.6, NH-CH₂; δ 147.9, C=N; δ 148.0, C=N; δ 174.4, C=O; δ 177.6, C=S; δ 178.5, C=S. MS: (+ve ion) *m*/*z* [(**L³**H₂) + H⁺]⁺ 361.4 (experimental), 361.1 (calculated), (-ve ion) *m*/*z* [(L³H₂) - H⁺]⁻ 359.4 (experimental), 359.1 (calculated).

### Reference Example 6

### Cu-diacetyl-bis-(N'⁴-hexanoic acid, N"⁴-methylthiosemicarbazone), Cu(L³)

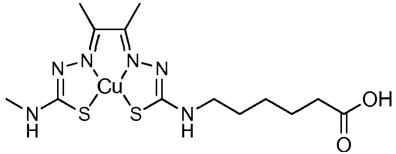

To a solution of the compound of example 5 (0.15 g, 0.4 mmol) in DMF (2 mL) stirring at room temperature was added Cu(OAc)₂.H₂O (0.08 g, 0.4 mmol). The red/brown solution was stirred at room temperature for 17 h. A brown solid was precipitated upon addition of water (40 mL) to the solution. The solid was collected by filtration, washed with water, ethanol and diethyl ether and dried to give **Cu(L³)** (0.16 g, 0.4 mmol, 88 %). MS: (+ve ion) **[Cu(L³)** + H⁺]⁺ *m*/*z* 100% 422.06471 (experimental), 422.06199 (calculated). RP HPLC: R_{T} = 11.023 min.

### Example 7 L⁴H₂

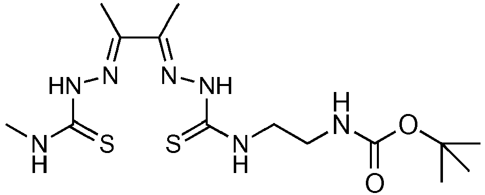

Following the same procedure employed in example 3, the product of example 2 (0.51 g, 1.9 mmol) in acetonitrile (8 mL) was added *tert*-butyl 2-aminoethylcarbamate (0.48 g, 3.0 mmol, 1.6 eq.). The resulting yellow suspension was heated at reflux for 3 h under an atmosphere of N₂ and was followed by TLC analysis (7.5% MeOH/CH₂Cl₂ v/v). Once the reaction was complete the resulting white suspension was cooled to room temperature and the white solid was collected by filtration, washed with acetonitrile (x1) and diethyl ether (x3) and dried to give H₂**L⁴** (0.59 g, 1.5 mmol, 81 %). *R_{f}* (7.5% MeOH/CH₂Cl₂ v/v) 0.5. (Found: C, 43.08; H, 6.88; N, 25.02; Calculated for C₁₄H₂₇N₇O₂S₂: C, 43.17; H, 6.99; N, 25.17). ¹H NMR (d₆-DMSO, 500 MHz): δ = 1.37, 9H, s, (CH₃)₃; 2.21, 3H, s, CH₃; 2.23, 3H, s, CH₃; 3.02, 3H, d, ³*J*_{HH} = 4.5 Hz, C*H*₃-NH; 3.18, 2H, q, ³*J*_{HH} = 5.5 Hz, C*H*₂NHC=O; 3.60, 2H, q, ³*J*_{HH} = 5.5 Hz, NHC*H*₂; 6.99, 1 H, t, ³*J*_{HH} = 5 Hz, N*H*C=O; 8.37, 1 H, q, ³*J*_{HH} = 4.5 Hz, N*H*-CH₃; 8.44, 1H, t, ³*J*_{HH} = 5 Hz, N*H*-CH₂; 10.24, 2H, br s, NH. ¹³C{¹H} NMR (125.7 MHz): δ = 11.7, CH₃; 11.8, CH₃; 28.2, (CH₃)₃; 31.2, NH-CH₃; 39.1, CH₂NHC=O; δ 44.5, NHCH₂; 77.9, C(CH₃)₃; 147.8, C=N; 148.3, C=N; 156.2, C=O; 178.1, C=S; 178.5, C=S. ESI-MS: (+ve ion) *m*/*z* 100% [M + H⁺] 390.17 (experimental), 390.17 (calculated).

### Reference Example 8 Cu^{II}(L⁴)

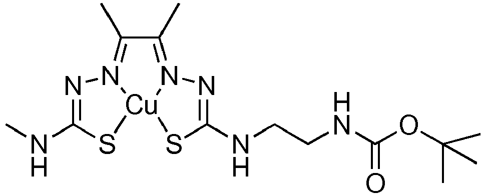

To a suspension of the compound of example 7 (0.08 g, 0.2 mmol) in ethanol (5 mL) was added Cu(OAc)₂.H₂O (0.04 g, 0.2 mmol). The red/brown solution was stirred at reflux for 4 h. The solvent was removed *in vacuo* and the brown residue was dissolved in dichloromethane (3 mL) and precipitated with hexane (30 mL). The solid was collected by filtration, washed with hexane and dried to give Cu^{II}(L⁴) (0.08 g, 0.18 mmol, 0.88%). (Found: C, 37.30; H, 5.60; N, 21.65; Calculated for CuC₁₄H₂₅N₇O₂S₂: C, 37.28; H, 5.59; N, 21.74). ESI-MS: (+ve ion) [M + H⁺] *m*/*z* 100% 451.09 (experimental), 451.08(calculated). RP HPLC (System E): R_{T} = 8.002 min. Crystals suitable for single crystal X-ray crystallography were grown from a concentrated solution in acetone.

### Example 9 [H₃L⁵][CF₃CO₂]

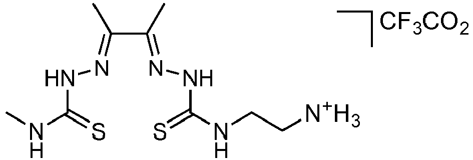

To a solution of trifluoroacetic acid (5 mL, 0 °C) cooled in an ice bath was added H₂**L⁴** (0.12 g, 0.3 mmol) in portions over 0.5 h under an atmosphere of nitrogen. The clear solution mixture was warmed to room temperature and stirred for 2 h. The solvent was removed to give yellow oil. Diethyl ether was added and a white solid precipitated, which was collected by filtration, washed with diethyl ether and dried to give [H₃**L⁵**][CF₃CO_{2]}.H₂O (0.096 g, 76 %). (Found: C, 31.65; H, 4.86; N, 22.80; Calculated for C₁₁H₂₀F₃N₇O₂S₂.H₂O: C, 31.35; H, 5.26; N, 23.26).¹H NMR (d₆-DMSO, 500 MHz): δ = 2.22, 6H, s, CH₃; 3.02, 3H, d, ³*J*_{HH} = 5.5 Hz, C*H₃*-NH; 3.05, 2H, t, ³*J*_{HH} = 7.5 Hz, CH₂; 3.83, 2H, q, ³*J*_{HH} = 7.5 Hz, CH₂; 7.83, 3H, br s, NH₃; 8.40, 1 H, m, N*H*-CH₃; 8.48, 1 H, t, ³*J*_{HH} = 7.5 Hz, N*H*-CH₂; 10.25, 1 H, br s, NH; 10.51, 1 H, br s, NH. ¹³C{¹H} NMR (125.7 MHz): *δ*= 11.8, CH₃; 11.9, CH₃; 31.2, NH-CH₃; 38.1, CH₂; δ 41.3, CH₂; 147.7, C=N; 148.9, C=N; 178.5, C=S. ESI-MS: (+ve ion) *m*/*z* 100% [M⁺] 290.12 (experimental), 290.12 (calculated). RP HPLC (System A): R_{T} = 7.497 min.

### Example 10 [Cu^{II}(H₂L⁵)][CF₃CO₂]₂

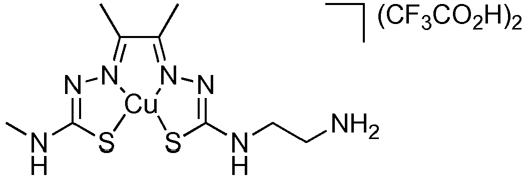

Following the same procedure employed for the synthesis of [H₃**L⁵**][CF₃CO_{2]}, Cu^{II}(**L⁴**) (0.04 g, 0.09 mmol) was used to prepare [Cu^{II}(H₂**L**⁵)][CF₃CO₂]₂ (0.04 g, 77 %). (Found: C, 26.80; H, 3.38; N, 16.65; Calculated for C₁₃H₁₉CuF₆N₇O₄S₂: C, 26.97; H, 3.31; N, 16.93). ESI-MS: (+ve ion) [M + H⁺] *m*/*z* 100% 351.04 (experimental), 351.04 (calculated), [M + 2H⁺] *m*/*z* 100% 176.02 (experimental), 176.02 (calculated). RP HPLC (System A): R_{T} = 7.227 min.

### Reference Example 11

### Solid-phase peptide synthesis

General Procedure: Side-chain protected BBN(7-14)-NH₂ (H-Gln(Trt)-Trp(Boc)-Ala-Val-Gly-His(Trt)-Leu-Met-NH-resin) was assembled on PAL-PEG resin (loading: 0.2 mmol/g) using standard Fmoc solid-phase peptide synthesis procedures. A solution of *bis*(thiosemicarbazone) or *bis*(thiosemicarbazonato)-copper(II) complex (2 to 3 equivalents) was pre-activated with HATU (2 to 3 equivalents) and DIPEA (4 to 6 equivalents) in DMF (1 mL) and added to the resin in a sintered glass funnel. The mixture was allowed to react for 1 h, with occasional stirring. The reaction solution was drained and the resin was washed with DMF (3 x 5 mL) and DCM (3 x 5 mL).

### Cleavage

Resin and protecting group cleavage was performed using a solution of TIPS/H₂O/TFA (2.5/2.5/95%) with 10 mL per 1 g of resin, which was shaken for 3 h. The solution was then filtered and sparged with a steady stream of N₂ to reduce the volume to 20%. Cold ether (40 mL) was then added to precipitate the peptide, which was then centrifuged for 4 min at 4000 rpm. The ether layer was decanted and the peptide was air-dried and solubilised in 50% acetonitrile/H₂O. The crude peptide was filtered and frozen with liquid N₂ and lyophilized.

### Peptide-bis(thiosemicarbazone) Purification

The crude peptide material was purified by semi-preparative reverse phase HPLC.

### Example 12

### Diacetyl-bis-(N'⁴-butyric acid, N"⁴-methylthiosemicarbazone)-BBN(7-14), L²H₂-BBN(7-14)

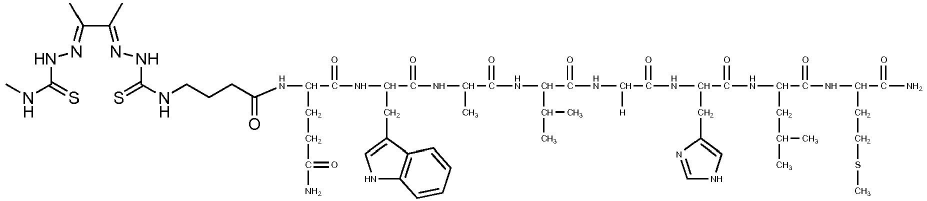

The Fmoc protected N terminus of bombesin (7-14) on PAL-PEG resin (0.3 g, 0.06 mmol) was cleaved. A solution of the compound of example 3 (35 mg, 0.13 mmol), HATU (50.7 mg, 0.13 mmol) and DIPEA (66 µL, 0.39 mmol) in DMF (1 mL) was added to the resin. The mixture was stirred and allowed to react for 1 h. The reaction solution was removed and the resin was washed with DMF (3 x 5 mL) and CH₂Cl₂ (3 x 5 mL). Resin and protecting group cleavage was performed as outlined above. The crude peptide material was purified by semi-preparative reverse phase HPLC employing a H₂O/CH₃CN (0 - 100% CH₃CN) linear gradient with a flow rate of 5 mL/min. Collected fractions containing pure **L²**H₂-BBN(7-14) were frozen with liquid N₂ and lyophilised. MS: (+ve ion) [**L²**H₂ + H⁺]⁺ *m*/*z* 1254.58109 (experimental), 1254.58107 (calculated), [**L²**H₂ + 2H⁺]²⁺ *m*/*z* 627.79415 (experimental), 627.79445 (calculated). RP HPLC: R_{T} = 12.518 min.

### Example 13

### Diacetyl-bis-(N'⁴-hexanoic acid, N"⁴-methylthiosemicarbazone)-BBN(7-14), L³H₂-BBN(7-14)

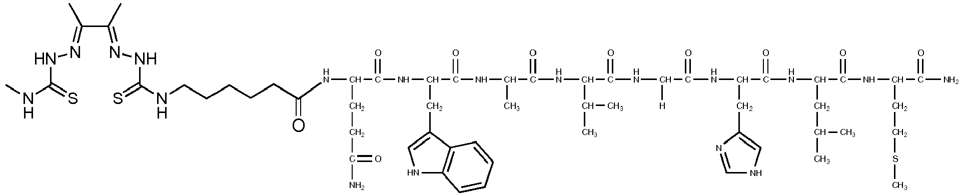

The Fmoc protected N terminus of bombesin (7-14) on PAL-PEG resin (0.24 g, 0.05 mmol) was cleaved. A solution of the compound of example 5 (36 mg, 0.1 mmol), HATU (39 mg, 0.1 mmol) and DIPEA (34 µL, 0.2 mmol) in DMF (1 mL) was added to the resin. The mixture was stirred and allowed to react for 2 h. The reaction solution was removed and the resin was washed with DMF (3 x 5 mL) and CH₂Cl₂ (3 x 5 mL). Resin and protecting group cleavage was performed as outlined above. The crude peptide material was purified by semi-preparative reverse phase HPLC employing a H₂O/CH₃CN (0 - 80% CH₃CN) linear gradient with a flow rate of 5 mL/min. Collected fractions containing pure **L³**H₂-BBN(7-14) were frozen with liquid N₂ and lyophilised (0.6 mg, 0.9%). MS: (+ve ion) [**L³**H₂ + H⁺]⁺ *m*/*z* 1282.61355 (experimental), 1282.61237 (calculated), [**L³**H₂ + 2H⁺]²⁺ *m*/*z* 641.81019 (experimental), 641.81001 (calculated). RP HPLC (H₂O/CH₃CN 0 - 80% CH₃CN): R_{T} = 19.097 min.

### Reference Example 14

### Cu-diacetyl-bis-(N'⁴-butanoic acid, N"⁴-methylthiosemicarbazone)-BBN(7-14), CuL²-BBN(7-14)

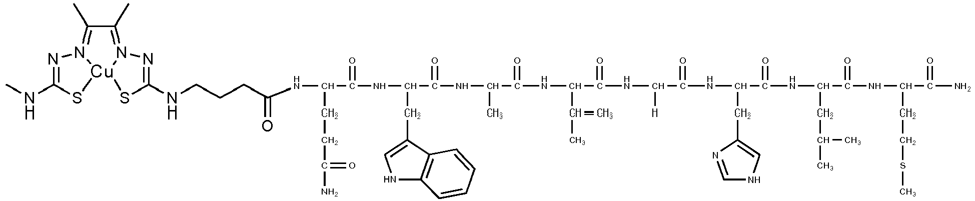

To BBN(7-14)-NH₂ on PAL-PEG resin (0.24 g) was added a solution of Cu^{II}(**L²**) (28 mg, 0.07 mmol), HATU (28 mg, 0.07 mmol) and DIPEA (25 µL, 0.14 mmol) in DMF (1 mL). ESI-MS: (+ve ion) [M + H⁺] *m*/*z* 1315.50 (experimental), 1315.50 (calculated), [M + 2H⁺] *m*/*z* 658.25 (experimental), 658.25 (calculated). RP HPLC (System D): R_{T} = 9.359 min.

### Reference Example 15

### Cu-diacetyl-bis-(N'⁴-hexanoic acid, N"⁴-methylthiosemicarbazone)-BBN(7-14), CuL³-BBN(7-14)

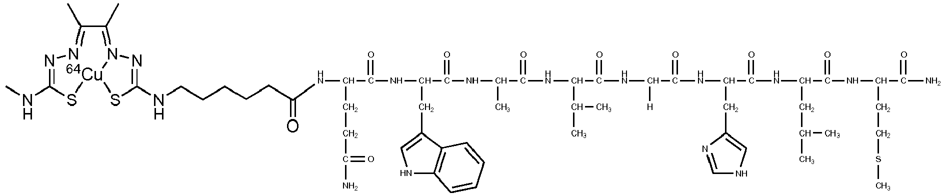

To BBN(7-14)-NH₂ on PAL-PEG resin (0.24 g, 0.2 mmol/g) was added a solution of Cu^{II}(**L³**) (31.5 mg, 0.08 mmol), HATU (30 mg, 0.08 mmol) and DIPEA (35 µL, 0.2 mmol) in DMF (1 mL). ESI-MS: (+ve ion) [M + H⁺] *m*/*z* 1343.53 (experimental), 1343.53 (calculated), [M + 2H⁺] *m*/*z* 672.27 (experimental), 672.27 (calculated). RP HPLC (System C): R_{T} = 13.858 min.

### Example 16

### Diacetyl-bis-(N'⁴-3-hydroxytyramine, N"⁴-methylthiosemicarbazone), L⁶H₂

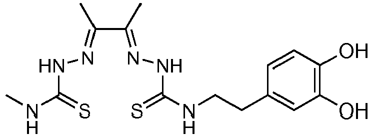

H₂**L⁶.** To a stirring suspension of H₂**L**¹ (0.16 g, 0.6 mmol) in acetonitrile (30 mL) was added 3-hydroxytyramine hydrochloride (0.22 g, 1.2 mmol, 2 eq.) and DIPEA (0.20 mL, 1.2 mmol). The resulting yellow suspension was heated at reflux for 22 h under an atmosphere of N₂. The resulting white suspension was cooled to room temperature and the white solid was collected by filtration, washed with HCl (3%, 3 x 3.5 mL), acetonitrile (x1) and diethyl ether (x3) and dried to give H₂**L⁶** (0.17 g, 0.45 mmol, 77 %). ¹H NMR (d₆-DMSO, 500 MHz): δ2.15, 3H, s, CH₃; δ2.20, 3H, s, CH₃; δ 2.70, 2H, t, ³J_{HH} = 7.5 Hz, NH-CH₂-C*H*₂; δ3.02, 3H, d, ³J_{HH} = 4 Hz, NH-CH₃; δ3.7, 2H, m, NH-C*H*₂; δ 6.49, 1 H, br, ArH; δ 6.64, 2H, br, ArH; δ 8.32, 1 H, br, NH-CH₂; δ 8.37, 1 H, br, N*H*-CH₃; δ8.66, 1 H, br, OH; δ 8.77, 1 H, br, OH; δ 10.21, 1 H, br, NH; δ 10.24, 1H, br, NH. ¹³C NMR (125 MHz): δ 11.6, CH₃; δ 11.7, CH₃; δ 31.2, NH-CH₃; δ 34.0, NH-CH₂-CH₂; δ 45.6, NH-CH₂; δ 115.6, C5; δ 115.9, C2; δ 119.2, C6; δ 129.8, C1; δ 143.6, C4; δ 145.2, C3; δ 147.8, C=N; δ 148.0, C=N; δ 177.6, C=S; δ 178.5, C=S. MS: (+ve ion) *m*/*z* [M + H⁺]⁺ 383.4 (experimental), 383.1 (calculated), (-ve ion) *m*/*z* [M - H⁺]⁻ 381.4 (experimental), 381.1 (calculated).

### Reference

### Example 17 Cu-diacetyl-bis-(N^{'4}-3-hydroxytyramine, N"⁴-methylthiosemi carbazone), Cu(L⁶)

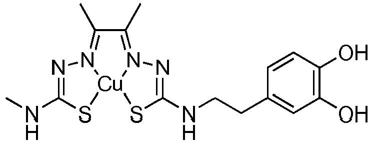

To a solution of **the compound of example 16** (0.07 g, 0.2 mmol) in DMF (2 mL) stirring at room temperature was added Cu(OAc)₂.H₂O (0.04 g, 0.2 mmol). The red/brown solution was stirred at room temperature for 22 h. A brown solid was precipitated upon addition of water (40 mL) to the solution. The solid was collected by filtration, washed with water, ethanol and diethyl ether and dried to give **Cu(L⁶)** (0.04 g, 44%). MS: (+ve ion) **[Cu(L⁶)** + H⁺]⁺ *m*/*z* 444.3 (experimental), 444.0 (calculated), (-ve ion) **[Cu(L⁶)** - H⁺]- *m*/*z* 442.3 (experimental), 442.0 (calculated) . RP HPLC: R_{T} = 11.230 min.

### Example 18

### Diacetyl-bis-(N'⁴-hexanoic amide 3-hydroxytyramine, N"⁴-methylthiosemicarbazone), L⁷H₂

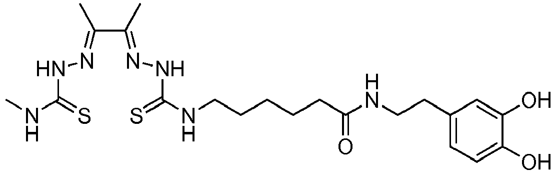

To a solution of the compound of example 5 (0.07 g, 0.2 mmol) and 3-hydroxytyramine hydrochloride (0.07 g, 0.4 mmol) in DMF (1mL) was added HATU (0.15 g, 0.4 mmol) and DIPEA (0.13 mL, 0.8 mmol). The reaction mixture was stirred at room temperature for 43 h whilst monitored by reverse-phase HPLC. Upon addition of water (30 mL) to the reaction mixture a white solid precipitated. The solid was collected by filtration, washed with water, acetonitrile (x3) and diethyl ether (x3) to give L⁷H₂ (0.7 g, 72%). ¹H NMR (d₆-DMSO, 500 MHz): δ [(L⁷H₂) + H⁺]⁺ *m*/*z* 496.5 (experimental), 496.1 (calculated), (-ve ion) [(**L**⁷H₂) - H⁺]- *m*/*z* 494.5 (experimental), 494.2 (calculated).

### Example 19

### Dimethylammonium diacetyl-bis-(N'⁴-sulfanilate, N"⁴-methylthiosemicarbazone), H₂N(CH₃)₂L⁸H₂

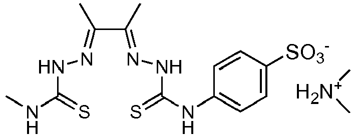

To a stirring suspension of **L**¹H₂ (0.32 g, 1.2 mmol) in acetonitrile (40 mL) was added sulfanilic acid (0.18 g, 1.1 mmol, 0.9 eq.). The resulting yellow suspension was heated at reflux for 4 h under an atmosphere of N₂. The resulting white suspension was cooled to room temperature and the white solid was collected by filtration, washed with acetonitrile (x1) and diethyl ether (x3) and dried to give **L⁸**H₂ (0.41 g, 77 %). ¹H NMR (d₆-DMSO, 500 MHz): δ2.25,3H, s, CH₃; δ 2.29,3H, s, CH₃; δ 2.55, 6H, t, ³J_{HH} = 5.5 Hz, H₂N⁺(C*H*₃); δ 3.04, 3H, d, ³J_{HH} = 4.5 Hz, NH-C*H*₃; δ 7.51-7.58, 4H, m, Ar-H; δ 8.17, 2H, br, *H*₂N⁺(CH₃)₂; δ 8.41, 1 H, d, ³J_{HH} = 4.5 Hz, N*H*-CH₃; δ 9.96, 1 H, s, NH; δ 10.30, 1 H, s, NH; δ 10.60, 1 H, s, NH. ¹³C NMR (125 MHz): δ 12.3, CH₃; δ 12.5, CH₃; δ 31.9, NH-CH₃; δ 34.8, H₂N⁺(CH₃)₂; δ 125.0, ArC; δ 125.9, ArC; δ 139.4, ArC; δ 145.9, ArC; δ 148.2, C=N; δ 149.8, C=N; δ 177.2, C=S; δ 178.9, C=S. MS: (-ve ion) *m*/*z* [(**L⁸**H₂) - H⁺]- 401 (experimental).

### Example 20

### Sodium diacetyl-bis-(N'4-sulfanilate, N"⁴-methylthiosemicarbazone), NaL⁸H₂

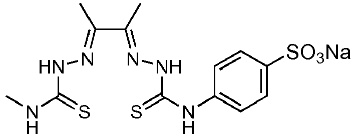

A solution of H₂N(CH₃)₂**L⁸**H₂ (0.41 g) in warm water (200 mL) was eluted through a Na⁺ Sephadex cation exchange column with a flow rate of 1 drop/2 seconds and collected. The column was eluted with a further 50 mL of water. The pale yellow eluent was collected and the solvent was removed under reduced pressure. The resulting powder was washed with acetonitrile (300 mL) and diethyl ether (300 mL) to give a gold powder (0.35 g, 87%). ¹H NMR (d₆-DMSO, 500 MHz): δ 2.25, 3H, s, CH₃; δ 2.29, 3H, s, CH₃; δ 3.03, 3H, d, ³J_{HH} = 4.5 Hz, NH-C*H*₃; δ 7.52-7.58, 4H, m, Ar-H; δ 8.41, 1H, d, ³J_{HH} = 4.5 Hz, N*H*-CH₃; δ 9.96, 1H, s, NH; δ 10.30, 1H, s, NH; δ 10.60, 1H, s, NH.¹³C NMR (125 MHz): δ 12.5, CH₃; δ 12.7, CH₃; δ 31.9, NH-CH₃; δ 125.2, ArC; δ 126.1, ArC; δ 139.6, ArC; δ 146.2, ArC; δ 148.5, C=N; δ 150.0, C=N; δ 177.5, C=S; δ 179.2, C=S. MS: (-ve ion) *m*/*z* [Na**L⁸**H₂ - H⁺]⁻ 401 (experimental).

### Reference Example 21

### Dimethylammonium Cu-diacetyl-bis-(N'4-sulfanilate, N"⁴-methylthiosemicarbazone), H₂N(CH₃)₂Cu(L⁸)

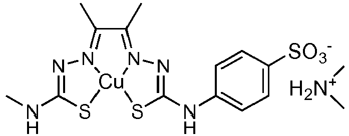

To a solution of H₂N(CH₃)₂**L⁸**H₂ (0.06 g, 0.1 mmol) in DMF (2 mL) stirring at room temperature was added Cu(OAc)₂.H₂O (0.03 g, 0.1 mmol). The red/brown solution was stirred at room temperature for 22 h. A brown solid was precipitated upon addition of diethyl ether (30 mL) to the solution. The solid was collected by filtration and washed with diethyl ether and dried to give H₂N(CH₃)₂Cu(**L⁸**) (0.03 g, 49%). MS: (+ve ion) [H₂N(CH₃)₂Cu(**L⁸**) + H⁺]⁺ *m*/*z* 463 (experimental).

### Reference Example 22

### Sodium Cu-diacetyl-bis-(N'⁴-sulfanilate, N"⁴-methylthiosemicarbazone), NaCu(L⁸₎

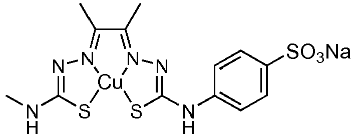

To a solution of Na**L⁸**H₂ (0.07 g, 0.2 mmol) in DMF (4 mL) stirring at room temperature was added Cu(OAc)₂.H₂O (0.03 g, 0.2 mmol). The red/brown solution was stirred at room temperature for 22 h. A brown solid was precipitated upon addition of diethyl ether (150 mL) to the solution. The solid was collected by filtration and washed with diethyl ether and dried to give NaCu(**L⁸**) (0.06 g, 80%). MS: (-ve ion) [NaCu(L⁸) - H⁺]⁺ *m*/*z* 462 (experimental).

### Example 23

### Potassium diacetyl-bis-(N'⁴-3-aminopropane-sulfonate, N"⁴-methylthiosemicarbazone), KL⁹H₂

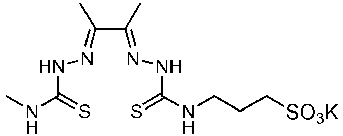

To a stirring suspension of **L**¹H₂ (0.07 g, 0.3 mmol) in acetonitrile (7 mL) was added 3-aminopropane-sulfonic acid (0.04 g, 0.3 mmol) and K₂CO₃ (0.04 g, 0.3 mmol). The resulting yellow suspension was heated at reflux for 20 h under an atmosphere of N₂. The resulting white suspension was cooled to room temperature and the white solid was collected by filtration, washed with acetonitrile (x1) and diethyl ether (x3) and dried to give an impure white powder (0.09 g). ¹H NMR (d₆-DMSO, 500 MHz): δ 1.84, 2H, m, CH₂-C*H*₂-CH₂; δ 2.18, 6H, s, CH₃; δ 2.45, 2H, t, CH₂-SO₃K; δ 3.01, 3H, d, NH-C*H*₃; δ 3.64, 2H, m, NH-C*H*₂; δ 8.21, 1H, br, NH; δ 8.51, 1H, br, NH.

### Example 24 (H₂L¹⁰)

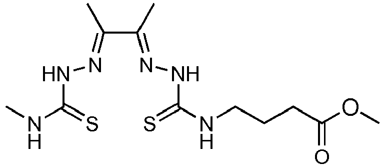

To a stirring suspension of H₂**L¹** (0.29 g, 1.1 mmol) in acetonitrile (30 mL) was added methyl-4-aminobutanoate hydrochloride (0.18 g, 1.2 mmol) and triethylamine (0.12 g, 1.2 mmol). The resulting yellow suspension was heated at reflux for 3 h under an atmosphere of N₂. The resulting light cream suspension was cooled to room temperature and the white solid was collected by filtration, washed with HCl (3 %, 3 x 3.5 mL), acetonitrile (x1) and diethyl ether (x3) and dried to give H₂**L¹⁰**(0.25 g, 0.7 mmol, 67 %). ¹H NMR (d₆ DMSO, 500 MHz): δ = 1.89, 2H, m, NH-CH₂-C*H*₂-CH₂; 2.21, 6H, s, CH₃; 2.34, 2H, t, NH-CH₂-CH₂-C*H*₂, ³*J*_{HH} = 7.5 Hz; 3.02, 3H, ³*J*_{HH} = 4.5 Hz; 3.58, 5H, m, NH-C*H*₂, OCH₃; 8.38, 1H, m, N*H*CH₃; 8.43, 1H, t, N*H*-CH₂, ³*J*_{HH} = 5.0 Hz; 10.20, 2H, s, NH. ESI-MS: (+ve ion) *m*/*z* 100% [M + H⁺] 347.4 (experimental), 347.1 (calculated), (-ve ion) *m*/*z* [M - H⁺] 345.4 (experimental), 345.1 (calculated).

### Example 25 (H₂L¹¹)

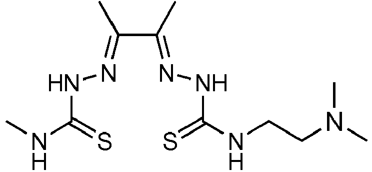

To a stirring suspension of H₂**L¹** (0.21 g, 0.85 mmol) in acetonitrile (30 mL) was added N-dimethylethylenediamine (0.01 g, 1.12 mmol). The resulting yellow suspension was heated at reflux for 6.5 h under an atmosphere of N₂. The resulting orange solution was cooled to room temperature resulting in the precipitation colourless crystals, which were collected by filtration, washed with acetonitrile (x1) and diethyl ether (x3) and dried to give H₂**L¹¹** (0.18 g, 0.55 mmol, 74 %). (Found: C, 41.69; H, 7.36; N, 30.74; Calculated for C₁₁H₂₃N₇S₂: C, 41.61; H, 7.30; N, 30.88). ¹H NMR (d₆ DMSO, 500 MHz): δ = 2.15, 3H, s, CH₃; 2.18, 6H, s, CH₃; 2.20, 3H, s, CH₃; 2.44, 2H, t, ³*J*_{HH} = 6.5 Hz, CH₂; 3.01, 3H, d, ³*J*_{HH} = 5 Hz, NH-C*H*₃; 3.60, 2H, q, NH-C*H*₂, ³*J*_{HH} = 5.5 Hz; 8.38-8.33, 2H, br, N*H*-CH₂, N*H*-CH₃; 10.25, 2H, br, NH. ESI-MS: (+ve ion) *m*/*z* 100% [M + H⁺] 318.4 (experimental), 318.2 (calculated), (-ve ion) *m*/*z* [M - H⁺] 316.4 (experimental), 316.1 (calculated).

### Example 26 (H₂L¹²)

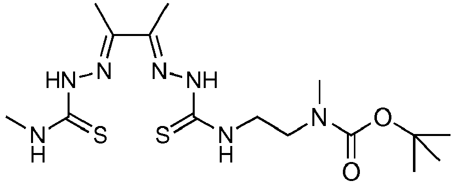

Following the same procedure employed for the synthesis of example 7 H₂**L¹** (0.55 g, 2.0 mmol) and (2-aminoethyl)methylcarbonic acid *tert*-butyl ester (0.42 g, 2.4 mmol) were used to prepare H₂**L¹²** (0.70 g, 86 %). (Found: C, 45.30; H, 7.17; N, 25.00; Calculated for C₁₅H₂₉N₇O₂S₂.0.5CH₃CN: C, 45.31; H, 7.25; N, 24.77). ¹H NMR (d₆ DMSO, 500 MHz): δ = 1.35, 9H, s, (CH₃)₃; 2.07, CH₃CN; 2.20, 6H, s, CH₃; 2.80-2.86, 3H, br, CH₃; 3.02, 3H, d, ³*J*_{HH} = 5 Hz, NH-C*H*₃; 3.41, 2H, br, CH₂; 3.65-3.74, 2H, br, CH₂; 8.45-8.36, 2H, br, NH; 10.26, 2H, s, NH. ESI-MS: (+ve ion) *m*/*z* 100% [M + H⁺] 404.5 (experimental), 404.2 (calculated), (-ve ion) *m*/*z* [M - H⁺] 402.4 (experimental), 402.2 (calculated).

### Example 27 (H₂L¹³)

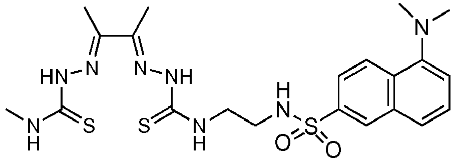

Following the same procedure employed in example 7, H₂**L¹** (0.15 g, 0.56 mmol) and (2-aminoethyl)dansylamide (0.16 g, 0.56 mmol) were used to prepare H₂**L¹³** (0.27 g, 92 %). ¹H NMR (d₆ acetone, 500 MHz): δ = 2.18, 3H, s, CH₃; 2.23, 3H, s, CH₃; 2.86, 6H, s, (CH₃)₂; 3.15, 3H, d, CH₃, ³*J*_{HH} = 5 Hz, NH-C*H*₃; 3.21, 2H, t, ³*J*_{HH} = 6 Hz, CH₂; 3.77, 2H, q, ³*J*_{HH} = 6 Hz, CH₂; 7.22, 1H, dd, ³*J*_{HH} = 7.5 Hz, ⁴*J*_{HH} = 0.5 Hz ArH; 7.55, 1H, dd, *J*_{HH} = 8.7, 7.6 Hz, ArH; 7.60, 1H, dd, *J*_{HH} = 8.5, 7.3 Hz; 8.22, 1H, dd, ³*J*_{HH} = 7.25 Hz, ⁴*J*_{HH} = 1.3 Hz, ArH; 8.27-8.31, 2H, br, NH; 8.36, 1H, dt, *J*_{HH} = 8.7, 0.9 Hz, ArH; 8.54, 1H, dt, *J*_{HH} = 8.5, 1.0 Hz . ESI-MS: (+ve ion) *m*/*z* 100% [M + H⁺] 523.4 (experimental), 523.2 (calculated), (-ve ion) *m*/*z* [M - H⁺] 521.4 (experimental), 521.2 (calculated).

### Example 28 (H₂L¹⁴)

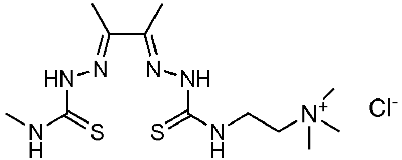

Following the same procedure employed for the synthesis of example 24 H₂**L¹** (0.20 g, 0.75 mmol), *N,N,N*-trimethylethane-1,2-diaminium hydrochloride (0.20 g, 1.13 mmol) and triethylamine (0.11 g, 1.13 mmol) were used to prepare H₂**L¹⁴** (0.19 g, 70 %). ¹H NMR (d₆ DMSO, 500 MHz): δ= 1.35, 9H, s, (CH₃)₃; 2.07, CH₃CN; 2.20, 6H, s, CH₃; 2.80-2.86, 3H, br, CH₃; 3.02, 3H, d, ³*J*_{HH} = 5 Hz, NH-C*H*₃; 3.41, 2H, br, CH₂; 3.65-3.74, 2H, br, CH₂; 8.45-8.36, 2H, br, NH; 10.26, 2H, s, NH.

### Example 29 (H₂L¹⁵)

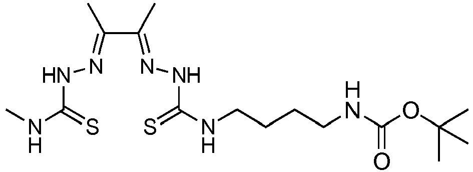

Following the same procedure employed in example 7 H₂**L¹** (0.20 g, 0.74 mmol) and *tert*-butyl 4-aminobutylcarbamate (0.21 g, 1.1 mmol) were used to prepare H₂**L¹⁵** (0.27 g, 87 %). ¹H NMR (d₆ DMSO, 500 MHz): δ = 1.35-1.42, 11H, m, (CH₃)₃, CH₂; 1.54, 2H, m, CH₂; 2.198, 3H, s, CH₃; 2.202, 3H, s, CH₃; 2.92, 2H, q, ³*J*_{HH} = 7 Hz, CH₂; 3.02, 3H, d, ³*J*_{HH} = 6 Hz, NH-C*H*₃; 3.55, 2H, q, ³*J*_{HH} = 8.5 Hz, CH₂; 6.80, 1 H, t, ³*J*_{HH} = 7 Hz, N*H*C=O; 8.35-8.40, 2H, m, NH; 10.13, 2H, br, NH.

### Example 30 (H₂L¹⁶)

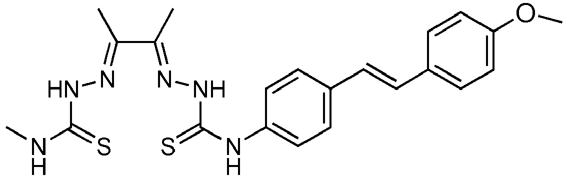

To a suspension of H₂**L¹** (0.15 g, 0.55 mmol) in acetonitrile (4 mL), (*E*)-4-amino-4'-methoxystilbene (0.12 g, 0.55 mmol) was added. The mixture was heated at reflux under an atmosphere of N₂ for 4 h. The mixture was cooled to rt., and Et₂O was added to precipitate the product. The yellow product was collected by filtration and washed with Et₂O (0.10 g, 41%). ¹H NMR (d₆- DMSO) (500 MHz): δ 2.26, 3H, s, CH₃; δ 2.29, 3H, s, CH₃; δ 3.03, 3H, d, ³J_{HH} = 4.5 Hz, NH-C*H₃*; δ 3.78, 3H, s, OCH₃; δ 6.94, 2H, d, ³J_{HH} = 9.0 Hz, ArH; δ 7.08, H, d, ³J_{HH} = 16.5 Hz, C=CH; δ 7.54, 4H, m, ArH; δ 7.60, 2H, d, ³J_{HH} = 9.0 Hz, ArH; δ 8.41, 1 H, d, ³J_{HH} = 4.5 Hz, CH₃-*NH*; δ 9.96, 1H, br, NH; δ 10.30, 1H, br, NH; δ 10.60, 1H, br, NH. MS: (+ve ion) ^{m}/_{z} 477.1 = [M + Na⁺]⁺.

### Reference Example 31 (Cu^{II}(L¹⁶)

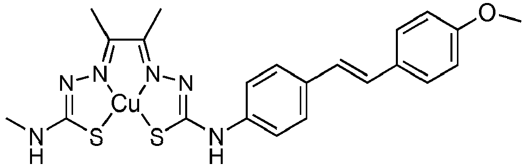

To a suspension of H₂**L¹⁶** (0.05 g, 0.10 mmol) in DMF (1 mL), copper acetate monohydrate (0.02 g, 0.12 mmol) was added. The mixture was stirred at rt. for 1 h. H₂O (3 mL) was added to precipitate the product and dissolve excess copper acetate. The mixture was centrifuged, the supernatant was removed while the pellet was washed with H₂O (x 3 times). The brown product was dried *in vacuo* (0.04 g, 81%). MS: (+ve ion) ^{m}/_{z} 516.3 = [M + H⁺]⁺. HPLC: *R*_{T} = 18.4 min. (Found: C, 51.25; H, 4.71; N, 15.98; Calc'd for CuC₂₂H₂₄N₆S₂O: C, 51.19; H, 4.69; N, 16.28).

### Example 32 (H₂L¹⁷)

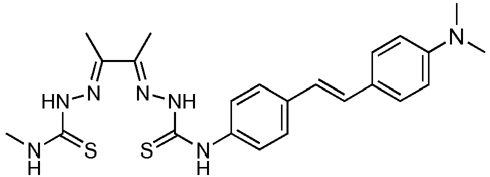

To a suspension of H₂**L¹** (0.20 g, 0.73 mmol) in acetonitrile (15 mL), (*E*)-4-amino-4'-dimethylaminostilbene (0.17 g, 0.73 mmol) was added. The mixture was heated at reflux under an atmosphere of N₂ for 16 h. The yellow-orange product was collected by filtration and washed with acetonitrile and Et₂O (0.21 g, 62%). ¹H NMR (d₆ - DMSO) (500 MHz): δ 2.25, 3H, s, CH₃; δ 2.29, 3H, s, CH₃; δ 2.93, 6H, s, N(CH₃)₂; δ 3.04, 3H, s, NH-C*H*₃; δ 6.72, 2H, d, ³J_{HH} = 8 Hz, ArH; δ 6.96, H, d, ³J_{HH} = 16.5 Hz, C=CH; δ 7.09, H, d, ³J_{HH} = 16.5 Hz, C=CH; δ 7.42-7.58, 6H, m, ArH; δ 8.41, H, br, NH; δ 9.95, H, br, NH; δ 10.30, H, br, NH; δ 10.57, H, br, NH. MS: (+ve ion) ^{m}/_{z} 468.20 = [M + H⁺]⁺.

### Reference Example 33 Cu^{II}(L¹⁷).

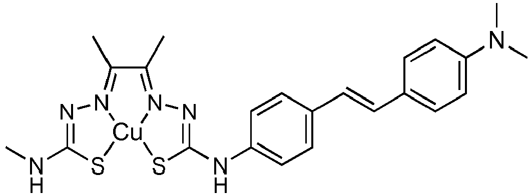

To a suspension of H₂**L¹⁷** (0.10 g, 0.21 mmol) in DMF (2 mL), copper acetate monohydrate (0.045 g, 0.23 mmol) was added. The mixture was stirred at rt. for 1 h. H₂O was added to precipitate the product and dissolve excess copper acetate. The mixture was centrifuged, the supernatant was removed while the pellet was washed with H₂O (x 3 times). The red-brown product was dried *in vacuo* (0.09 g, 79%). MS: (+ve ion) ^{m}/_{z} 529.11 = [M + H⁺]⁺. HPLC: *R*_{T} = 13.6 min.

### Example 34 (H₂L¹⁸)

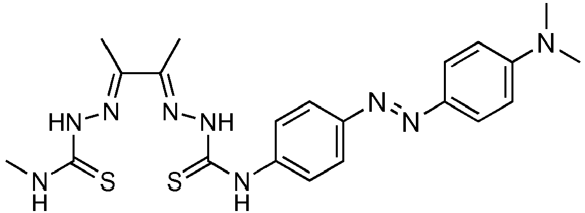

To a suspension of H₂**L¹** (0.10 g, 0.36 mmol) in acetonitrile (10 mL), *N,N-*dimethyl-4,4'-azodianiline (0.09 g, 0.36 mmol) was added. The mixture was heated at reflux under an atmosphere of N₂ for 16 h. The red-brown product was collected by filtration and washed with acetonitrile and Et₂O (0.065 g, 38%). ¹H NMR (d₆- DMSO) (500 MHz): δ 2.23, 3H, s, CH₃; δ 2.27, 3H, s, CH₃; δ 3.04, 3H, d, ³J_{HH} = 4.5 Hz, NH-C*H₃;* δ 3.06, 6H, s, N(CH₃)₂; δ 6.83, 2H, d, ³J_{HH} = 9.5 Hz, ArH; δ 7.77-7.83, 6H, m, ArH; δ 8.41, H, br, NH; δ 10.08, H, br, NH. MS: (+ve ion) ^{m}/_{z} 470.19 = [M + H⁺]⁺. HPLC: *R*_{T} = 15.4 min.

### Reference Example 35 Cu^{II}(L¹⁸).

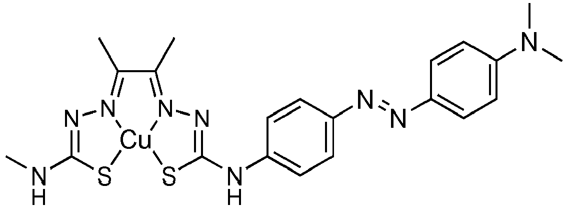

To a suspension of H₂**L¹⁸** (0.04 g, 0.085 mmol) in DMF (1 mL), copper acetate monohydrate (0.018 g, 0.089 mmol) was added. The mixture was stirred at rt. for 1 h. H₂O was added to precipitate the product and dissolve excess copper acetate. The mixture was centrifuged, the supernatant was removed while the pellet was washed with H₂O (x 3 times). The red-brown product was dried *in vacuo* (0.02 g, 49%). MS: (+ve ion) ^{m}/_{z} 531.11 = [M + H⁺]⁺. HPLC: *R*_{T} = 13.6 min.

### Example 36 (H₂L¹⁹)

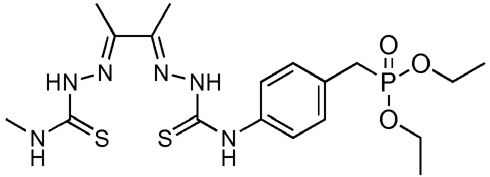

To a suspension of H₂**L¹** (0.102g, 0.37 mmol) in acetonitrile (10 mL), diethyl-4-aminobenzyl phosphonate (0.09 g, 0.37 mmol) was added. The mixture was heated at reflux under an atmosphere of N₂ for 16 h. The yellow product was collected by filtration and washed with acetonitrile and Et₂O (0.066 g, 37%). ¹H NMR (d₆- DMSO) (500 MHz): δ1.19, 6H, t, CH₂-C*H*₃; δ 2.24, 3H, s, CH₃; δ 2.28, 3H, s, CH₃; δ 3.03, 3H, d, ³J_{HH} = 4.5 Hz, NH-C*H*₃; δ 3.20, 2H, d, ³J_{HH} = 25 Hz, P-C*H*₂; δ 3.93-3.99, 4H, m, O-C*H*₂; δ 7.25, 2H, d, ³J_{HH} = 5 Hz, ArH; δ 7.49, 2H, d, ³J_{HH} = 8.5 Hz, ArH; δ 8.40, 1 H, d, ³J_{HH} = 4.5 Hz, CH₃-*NH*; δ 9.92, 1 H, br, NH; δ 10.34, 2H, br, NH. MS: (+ve ion) ^{m}/_{z} 473.15 = [M + H⁺]⁺.

### Example 37 [Me₂NH₂][H₂L²⁰]

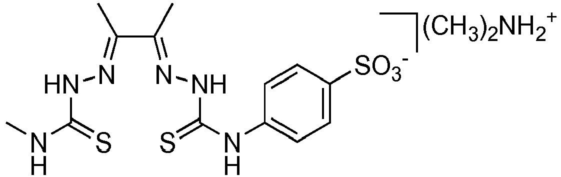

H₂**L¹** (0.36 g, 1.3 mmol) and sulfanilic acid (0.20 g, 1.2 mmol) were suspended in acetonitrile (40 mL) and the mixture heated under reflux at 98°C under nitrogen for 4 h. The hot mixture was filtered to provide a pale yellow solid which was washed with MeCN and Et₂O and dried in air (0.44 g, 76 %). ¹H NMR, (CD₃)₂SO). δ = 2.25 (s, 3H); 2.29 (s, 3H); 2.53-2.57 (t, *J* = 5.5 Hz, 6H); 3.02-3.06 (d, *J* = 4.5 Hz, 3H); 7.50-7.54 (m, 2H); 7.56-7.60 (m, 2H); 7.97-8.36 (br, 1 H); 8.39-8.43 (q, *J* = 4 Hz, 1 H); 9.96 (s, 1H); 10.30 (s, 1 H); 10.59 (s, 1 H). ¹³C NMR, (CD₃)₂SO δ = 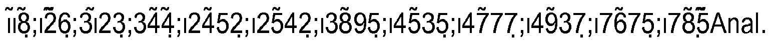 Found: C, 39.33; H, 5.69; N, 21.27; calc for [C₁₅H₂₅N₇O₃S₃]: C, 40.25; H, 5.63; N, 21.91; calc for ([C₁₅H₂₅N₇O₃S₃].0.5H₂O) - C, 39.46; H, 5.74; N, 21.47Anal. Found: C, 39.33; H, 5.69; N, 21.27; calc for [C₁₅H₂₅N₇O₃S₃]: C, 40.25; H, 5.63; N, 21.91; calc for ([C₁₅H₂₅N₇O₃S₃].0.5H₂O) - C, 39.46; H, 5.74; N, 21.47.

### Reference Example 38 [Me₂NH₂][Zn^{II}(L²⁰)].

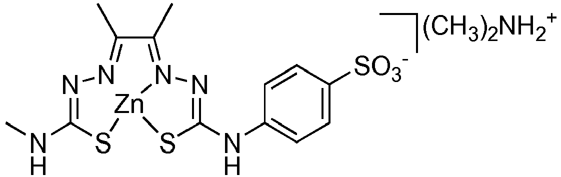

A solution of Zn(MeCO₂)₂.2H₂O (0.05 g, 0.22 mmol) in Dmf (1 mL) was added dropwise to the compound of example 37 (0.10 g, 0.22 mmol) in Dmf (2 mL). The solution was stirred under nitrogen at room temperature for 3 h. Et₂O (60 mL) was added dropwise precipitating a yellow solid which was collected by filtration, washed with Et₂O and dried under high vacuum (0.1 g, 89 %). ¹H NMR, (CD₃)₂SO): δ 2.25 (s, 3H); δ 2.31 (s, 3H); δ 2.55 (s, 6H); δ 2.80-2.90 (bs, 3H); δ 7.43-7.51 (m, 2H); δ 7.69-7.78; δ 8.05-8.40; δ 9.39 (s, 1H). Anal. Found C, 35.31; H, 4.59; N, 18.82; calc for C₁₅H₂₃ZnN₇O₃S₃ C, 35.26; H, 4.54; N, 19.19.

### Example 39 (H₂L²¹)

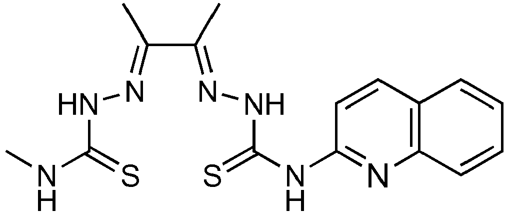

2-Aminoquinoline (200 mg, 1.40 mmol) was dissolved in dry, degassed acetonitrile (20 mL) to which was added a suspension of H₂**L¹** (360 mg, 1.2 mmol). The reaction was heated at reflux for 12 h. On cooling to room temperature, a light beige precipitate was collected, washed with ether (3 x 10 mL) and air-dried (110 mg, 25%). ¹H NMR (500 MHz; DMSO-*d₆*): δ/ppm 11.40 (s, 1 H, N-N*H*-C=S), 10.33 (s, 1 H, N-N*H*-C=S), 8.53-8.50 (m, 1 H, CH₃-N*H*-C=S), 8.40-8.36 (m, 1 H, ArH), 8.31-8.28 (m, 1 H, Q-N*H*-C=S), 7.93-7.91 (m, 1 H, ArH), 7.80-7.73 (m, 2H, ArH), 7.54-7.51 (m, 1 H, ArH), 7.45-7.42 (m, 1 H, ArH), 3.09-3.04 (m, 3H, C*H*₃-NH-C=S), 2.56 (s, 3H, C*H*₃), 2.28 (s, 3H, C*H*₃). MS (ES⁺): *m*/*z* (calcd) 374.12 (374.12) [M + H⁺].

### Example 40 (H₂L²²)

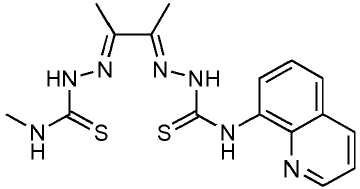

8-Aminoquinoline (200 mg, 1.40 mmol) was dissolved in dry, degassed acetonitrile (20 mL) to which was added a suspension of H₂**L¹** (360 mg, 1.2 mmol). The reaction was heated at reflux for 12 h. On cooling to room temperature, a crystalline off white precipitate was collected, washed with ether (3 x 10 mL) and air-dried (365 mg, 82%). ¹H NMR (500 MHz; DMSO-*d₆*): δ/ppm 12.38 (s, 1H, N-N*H-*C=S), 11.08 (s, 1 H, Q-N*H*-C=S), 10.37 (s, 1 H, N-N*H*-C=S), 9.55 (dd, ³*J*_{HH}= 7.7, ⁴*J*_{HH} = 1.0, 1 H, ArH), 8.84 (dd, ³*J*_{HH} = 4.2, ⁴*J*_{HH} = 1.7, 1 H, ArH), 8.48-45 (m, 2H, CH₃-N*H-*C=S & ArH), 7.74 (dd, ³*J*_{HH} = 8.3, ⁴*J*_{HH} = 0.9, 1 H, ArH), 7.70 (dd, ³*J*_{HH} = 8.2, ⁴*J*_{HH} = 4.2, 1 H, ArH), 7.64 (t, ³*J*_{HH} = 8.0, 1H, ArH), 3.05 (m, 3H, C*H*₃-NH-C=S), 2.48 (s, 3H, C*H*₃), 2.36 (s, 3H, C*H*₃). MS (ES⁺): *m*/*z* (calcd) 374.12 (374.12) [M + H⁺]. Following the procedures described above the following compounds should also be able to be made.

### Example 41 (H₂L²³)

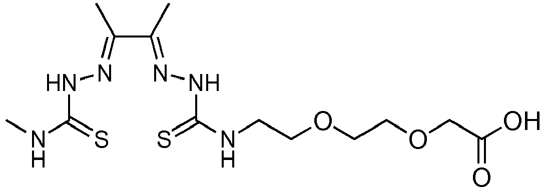

To a stirring suspension of H₂**L¹** (0.28 g, 1.0 mmol) in acetonitrile will be added (2-(2-aminoethoxy)ethoxy)acetic acid. The resulting mixture will be heated at reflux under an atmosphere of N₂ followed by cooling to room temperature and work up.

### Example 42 (H₂L²⁴)

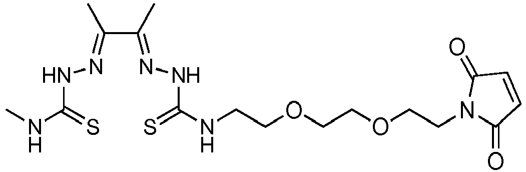

To a stirring suspension of H₂**L¹** in acetonitrile will be added N, N-maleimidoyl-N'-lipoyl-2,2'-(ethylene-1,2-dioxy)bisethylamide. The resulting mixture will be heated at reflux under an atmosphere of N₂ followed by cooling to room temperature and work up.

### Example 43 (H₂L²⁵)

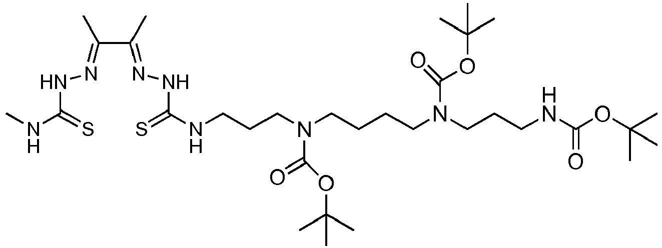

To a stirring suspension of H₂**L¹** in acetonitrile will be added tri-tert-butyl carbonyl spermine. The resulting mixture will be heated at reflux under an atmosphere of N₂ followed by cooling to room temperature and work up.

### Example 44 (H₂L²⁶)

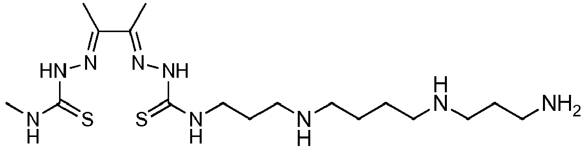

Following the same procedure employed for the synthesis of example 9 **[H₃L⁵][CF₃CO₂]**, H₂**L¹** and H₂**L²⁵** will be used to prepare H₂**L²⁶**.

### Example 45 (H₂L²⁷)

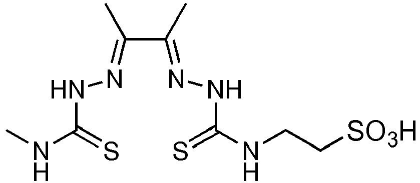

To a stirring suspension of H₂**L¹** in acetonitrile will be added 2-aminoethanesulfonic acid. The resulting mixture will be heated at reflux under an atmosphere of N₂ followed by cooling to room temperature and work up.

### Example 46 (H₂L²⁸)

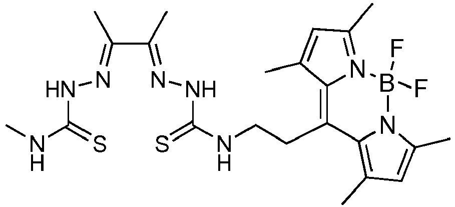

To a stirring suspension of H₂**L¹** in acetonitrile will be added (2-aminoethyl)bodipy. The resulting mixture will be heated at reflux under an atmosphere of N₂ followed by cooling to room temperature and work up.

### Example 47 (H₂L²⁹)

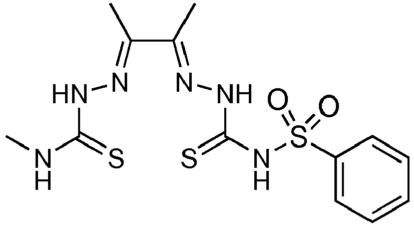

To a stirring suspension of H₂**L¹** in acetonitrile will be added benzenesulfonamide. The resulting mixture will be heated at reflux under an atmosphere of N₂ followed by cooling to room temperature and work up.

### Example 48 (H₂L³⁰)

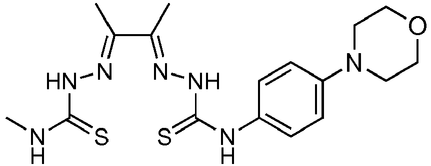

To a stirring suspension of H₂**L¹** in acetonitrile will be added 4-morpholinobenzenamine. The resulting mixture will be heated at reflux under an atmosphere of N₂ followed by cooling to room temperature and work up.

### Example 49 (H₂L³¹)

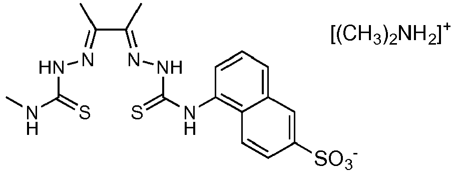

To a stirring suspension of H₂**L¹** in acetonitrile will be added 5-aminonaphthalene-2-sulfonic acid. The resulting mixture will be heated at reflux under an atmosphere of N₂ followed by cooling to room temperature and work up.

### Example 50 (H₂L³²)

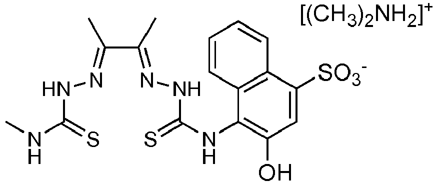

To a stirring suspension of H₂**L¹** in acetonitrile will be added 4-amino-3-hydroxynaphthalene-1-sulfonic acid. The resulting mixture will be heated at reflux under an atmosphere of N₂ followed by cooling to room temperature and work up.

### Example 51 (H₂L³³)

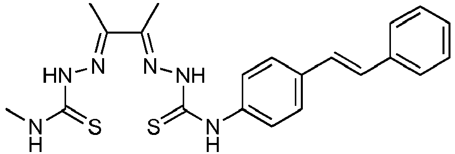

To a stirring suspension of H₂L¹ in acetonitrile will be added (*E*)4-aminostilbene. The resulting mixture will be heated at reflux under an atmosphere of N₂ followed by cooling to room temperature and work up.

### Example 52 (H₂L³⁴)

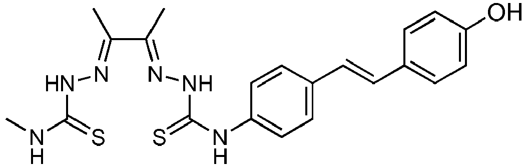

To a stirring suspension of H₂**L¹** in acetonitrile will be added (*E*)4-amino-4'-hydroxystilbene. The resulting mixture will be heated at reflux under an atmosphere of N₂ followed by cooling to room temperature and work up.

### Example 53 (H₂L³⁵)

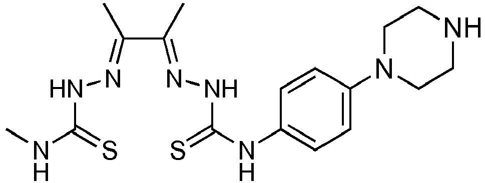

To a stirring suspension of H₂**L¹** in acetonitrile will be added 4-(piperazin-1-yl)benzenamine. The resulting mixture will be heated at reflux under an atmosphere of N₂ followed by cooling to room temperature and work up.

### Example 54 (H₂L³⁶)

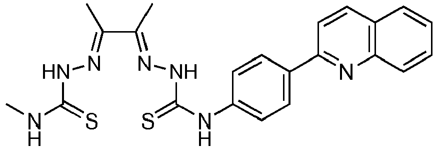

To a stirring suspension of H₂**L¹** in acetonitrile will be added 2-(4-aminophenyl)quinoline hydrochloride and triethylamine. The resulting mixture will be heated at reflux under an atmosphere of N₂ followed by cooling to room temperature and work up.

### Reference Example 55 X-ray Crystallographic studies

Crystals of H₂**L¹** (example 2) and [Cu^{II}(**L⁴**).(CH₃)₂CO (example 8) respectively were mounted in low temperature oil then flash cooled to 130 K using an Oxford low temperature device. Intensity data were collected at 130 K with an Oxford XCalibur X-ray diffractometer with Sapphire CCD detector using Cu-Kα radiation (graphite crystal monochromator λ = 1.54184 A). Data were reduced and corrected for absorption. The structures were solved by direct methods and difference fourier synthesis using the SHELX suite of programs² as implemented within the WINGX software.² Thermal ellipsoid plots were generated using the program ORTEP-3 integrated within the WINGX suite of programs. An ORTEP-3 representation of the X-ray crystal structure of H₂**L¹** is shown below (hydrogen atoms omitted for clarity except hydrogen atoms bound to nitrogen). The structure shows that in the solid state the molecule adopts an (*E*, *E*)-configuration about the imine double bonds and an s*-trans* (antiperiplanar) conformation about the C(5)-C(4) bond much like the ligand H₂atsm. There are small differences in the bond lengths between the thiosemicarbazone arms. The arm bearing a dimethyl substituent has a slightly shorter C(3)-S(1) (1.6802(19) A) bond length and a longer C(3)-N(1) (1.341(2) Å) bond length than the arm with a single methyl substituent (1.693(2) and 1.323(3) Å respectively). The bond lengths indicate that there is extensive delocalization throughout the molecule but the tautomeric form shown dominates.

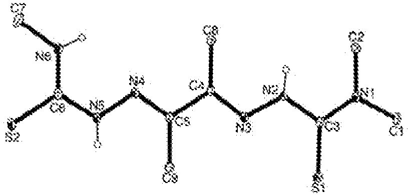

Crystals of Cu^{II}(**L⁴**) suitable for X-ray crystal structure determination were grown from a concentrated solution of the complex in acetone. An ORTEP-3 representation of the X-ray crystal structure of Cu^{II}(**L⁴**) is shown below(hydrogen atoms omitted for clarity except hydrogen atoms bound to nitrogen). The Cu atom is four coordinate and sits 0.052 A out of the plane of the N₂S₂ square planar donor system. The Cu-N and Cu-S bond distances are similar to Cu^{II}(atsm) (Table 1).The distortion from ideal square planar geometry is highlighted by the bond angle S(1)-Cu(1)-S(2) of 109.23(4)°.

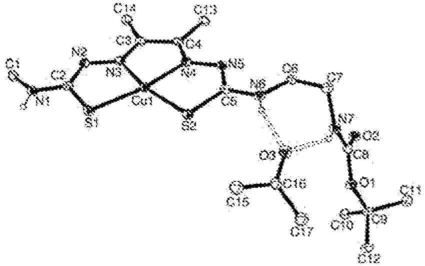

The ligand is dianionic as shown by the increase in the C-S bond distances (1.763(4) and 1.759(3) A) compared to the neutral proligand H₂**L¹** (1.6802(19) and 1.693(2) A). The ligand adopts an s-*cis* (synperiplanar) conformation about the C(3)-C(4) bond upon complexation. The flexibility of the sidechain is demonstrated by the dihedral angle defined by the atoms N(6)-C(6)-C(7)-N(7) of 60.02°. This angle is influenced by the packing and hydrogen bonding to a solvent molecule of acetone and a second molecule of Cu^{II}(**L⁴**). One of the NH groups forms a hydrogen bond to the carbamate O atom of a second molecule of the Cu complex (N(1)∧O(2)' 2.876 Å, N(1)-H(1)∧O(2)' 165.34°, symmetry operator x+1, y, z-1). The other NH groups form hydrogen bonds to the O atom of the molecule of solvent acetone. The stronger of the hydrogen bonds involves the carbamate NH group (N(7)AO(3) 2.866 A, N(7)-H(7)AO(3) 150.28°) while the weaker hydrogen bond involves the thioamide NH group (N(6)AO(3) 3.166 Å, N(6)-H(6)AO(3) 164.03°).

**Table 1 Selected bond distances (Å) and angles (°) in Cu^{II}(L⁴).(CH₃)₂CO**

| Cu^{II}(L**⁴**).(CH₃)₂CO | | | |
|---|---|---|---|
| Cu1-N3 | 1.965(3) | S1-Cu1-S2 | 109.23(4) |
| Cu1-N4 | 1.965(3) | N3-Cu1-N4 | 80.67(12) |
| Cu1-S1 | 2.2396(10)- | N3-Cu1-S1 | 84.81(8) |
| Cu1-S2 | 2.2517(10) | N4-Cu1-S2 | 85.16(9) |

**Table 2 Crystallographic data.**

| Crystal identification | H₂**L1** | Cu^{II}(**L⁴**).(CH₃)₂CO |
|---|---|---|
| Chemical formula | C₉H₁₈N₆S₂ | C₁₇H₃₁CuN₇O₃S₂ |
| *M* | 274.41 | 509.15 |
| Crystal system | monoclinic | triclinic |
| Space group | P 2₁/c | P-1 |
| *a*/*Å* | 15.4720(2) | 9.0904(6) |
| *b*/Å | 6.81020(10) | 11.5022(9) |
| *c*/Å | 13.66470(10) | 12.4161(9) |
| α/° | 90.00 | 78.916(6) |
| β/° | 109.1160(10) | 72.470(6) |
| γ/° | 90.00 | 85.134(6) |
| *V*/*Å*³ | 1360.42(3) | 1214.34(15) |
| *Z* | 4 | 4 |
| Independent Reflections | 2679 | 4699 |
| *R*ᵢₙₜ | 0.0298 | 0.0402 |
| *R (I* > *2s(I))* | 0.0400 | 0.0488 |
| *wR* (*all data)* | 0.1124 | 0.1502 |

### Reference Example 56 Electrochemistry

Recent studies have shown that *in vivo* stability of bifunctional chelators of copper is not only dependent on the thermodynamic stability and kinetic inertness of the complex but also the susceptibility to reduction of Cu(II) to Cu(I). Intracellular reductants with thiols, such as cysteine-rich metallothioneins, may act as effective reductants of Cu(II) and scavengers of the labile Cu(I) ions. Reduction potentials have been hypothesized to be a good way of predicting *in vivo* stability where complexes that are harder to reduce are more stable. The stability of the resulting Cu(I) ion upon reduction has been shown to be influential in the hypoxia selectivity exhibited by the *bis*(thiosemicarbazonato)-copper(II) complex Cu^{II}(atsm). It is proposed that this neutral complex can cross cell membranes and is retained selectively in cells experiencing hypoxia but is able to be 'washed out' of cells under normal oxygen conditions. The alkyl substituents on the diimine backbone seem to be of most importance regarding redox potentials whereas substitution at the N⁴-terminus does not influence redox potentials significantly. The complex Cu^{II}(atsm), undergoes a quasi-reversible reduction at *E*_{*1*/}*₂* = -0.63 V *(vs.* SCE, where *E*_{1/2} = [*E*_{pc} + Eₚₐ]/2 and Fc/Fc⁺ = 0.54 V) in anhydrous DMF at a glassy carbon working electrode. Cyclic voltammetry of the new Cu(II) complexes indicated that the structural change at the N⁴-terminus has not altered the electrochemistry of the parent complex Cu^{II}(atsm) significantly (Table 3).

**Table 3**

| Table of half-wave potentials and peak separations of the cyclic voltammograms. | | | | |
|---|---|---|---|---|
| Scan rate 0.1 V s⁻¹. Potentials are quoted relative to a SCE. | | | | |
| Compound | Cu^{II}/Cu^{I} E_{1/2} (mV) | Cu^{II}/Cu^{I} Eₚₐ-E_{pc} (mV) | Cu^{III}/Cu^{II} E_{1/2}(mV) | Cu^{III}/Cu^{II} Eₚₐ-E_{pc} (mV) |
| Cu^{II}(atsm) | -0.63 | 102 | 0.75 | 94 |
| Cu^{II}(**L²**) | -0.63 | 99 | 0.75 | 104 |
| Cu^{II}(**L³**) | -0.63 | 101 | 0.74 | 101 |
| Cu^{II}(**L⁴**) | -0.61 | 113 | 0.76 | 113 |
| Cu^{II}(**L⁵**) | -0.58 | 101 | 0.78 | 91 |

Cu^{II}(**L**³) for example, in DMF at a glassy carbon electrode, has a quasi-reversible reduction at *E*_{*1*/}*₂* = -0.63 V with an anodic to cathodic peak separation of 101 mV which was attributed to a Cu(II)/Cu(I) reduction process (see below). Under the same conditions the ferrocene/ferrocinium couple has a peak separation of 104 mV. There was also a quasi-reversible process at *E*_{*1*/}*₂* = 0.74 V (*vs.* SCE) with an anodic to cathodic peak separation of 101 mV, which is tentatively attributed to a Cu(II)/Cu(III) process.

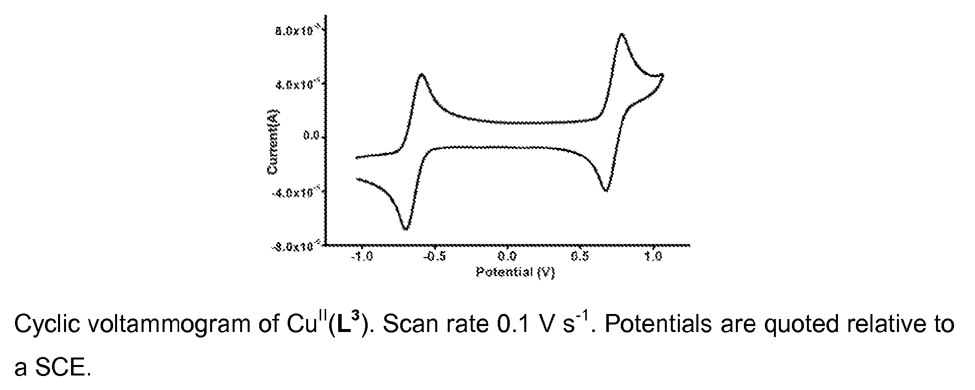

The isolated dicationic complex [Cu^{II}(H₂**L⁵**)]²⁺ exhibits an electrochemically irreversible reduction, however the addition of triethylamine to the analyte solution to neutralize the complex to give [Cu^{II}(**L⁵**)] restores the expected electrochemistry (see below). Protonation significantly alters the reduction potential of the Cu(II) complex, which has also been observed with a cationic thiosemicarbazone-pyridylhydrazine Cu(II) complex.³¹ As in the present case deprotonation of that complex with a base restored the quasi-reversibility of the Cu(II)/Cu(I) reduction process.

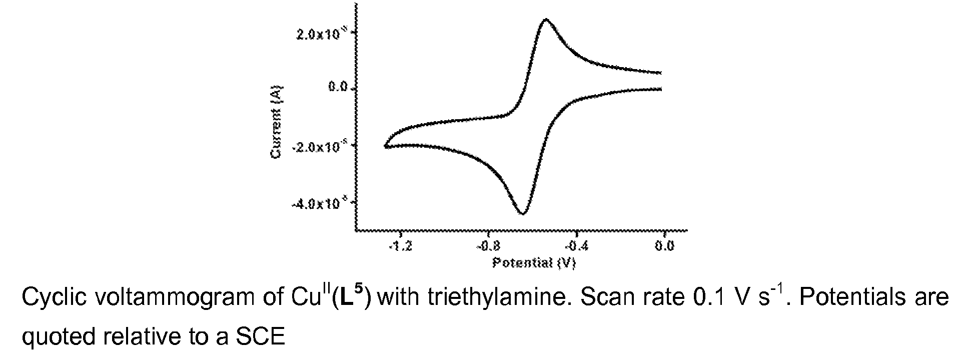

### Reference Example 57 ⁶⁴Cu Radiolabelling studies

⁶⁴CuCl₂ (1.88 GBq/mL, pH 1) was purchased from ANSTO radiopharmaceuticals and industrials (ARI), Lucas Heights, NSW, Australia. The radionuclidic purity at calibration {(⁶⁴Cu)/(⁶⁷Cu)} was 100% and the radiochemical purity as Cu(II) was 100%. The chemical purity of copper, zinc and iron were 1.1 µg/mL, 0.9 µg/mL and 10 µg/mL respectively.

### General Procedure

An aliquot of ⁶⁴CuCl₂ (20 µL, ~35 MBq, pH 1.0) was added to a solution containing the ligand (10 µL, 1 mg/mL DMSO), sodium acetate (90 µL, 0.1 M) and milliQ water (390 µL). The reaction was left for 30 min at room temperature before 100 µL of the reaction solution was injected onto a reverse-phase C18 analytical HPLC column. A DMSO solution of the 'cold' copper complex (1 mg/mL) was injected (8 µL) under the same conditions (λ = 275 nm) to verify the identity of the radiolabelled complex.

The retention times are as follows: ⁶⁴Cu^{II}(**L**³-BBN(7-14)-NH₂) (example 15): RP-HPLC (System C) R_{T}: 13.625 min and Cu^{II}(**L**³-BBN(7-14)-NH₂) (example 15), R_{T}: 13.858 min. ⁶⁴Cu^{II}(**L**²-BBN(7-14)-NH₂) (example 14): RP-HPLC (System D) R_{T}: 9.377 min and Cu^{II}(**L**²-BBN(7-14)-NH₂) (example 14), R_{T}: 9.359 min. ⁶⁴Cu^{II}(**L**³) : RP-HPLC (System C) R_{T}: 13.278 min and Cu^{II}(**L**³), R_{T}: 13.197 min. ⁶⁴Cu^{II}(**L**²):RP-HPLC (System C) R_{T}: 11.446 min and Cu^{II}(**L**²), R_{T}: 11.882 min. ⁶⁴Cu^{II}(**L**⁵): RP-HPLC (System E) R_{T}: 7.251 min and Cu^{II}(L⁵), R_{T}: 6.601 min. ⁶⁴Cu^{II}(**L**⁴): RP-HPLC (System E) R_{T}: 8.340 min and Cu^{II}(**L**⁴), R_{T}: 8.002 min.

Finally, it will be appreciated that various modifications and variations of the methods and compositions of the invention described herein will be apparent to those skilled in the art. Although the invention has been described in connection with specific preferred embodiments, it should be understood that the invention as claimed should not be unduly limited to such specific embodiments.

## Claims

1. A method of making a compound of the formula (I): 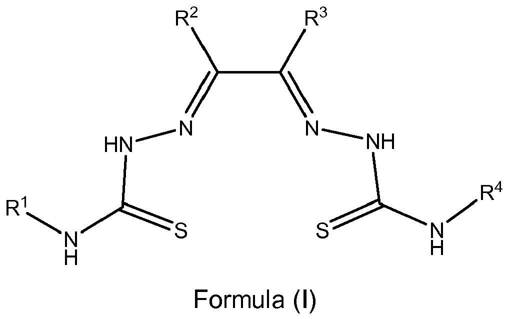 wherein
R¹ is selected from the group consisting of C₁-C₁₂alkyl, C₂-C₁₂alkenyl, C₂-C₁₂alkynyl, C₂-C₁₂heteroalkyl, C₃-C₁₂cycloalkyl, C₂-C₁₂heterocycloalkyl, C₆-C₁₈aryl, and C₁-C₁₈heteroaryl;
R⁴ is selected from the group consisting of optionally substituted C₁-C₁₂alkyl, optionally substituted C₂-C₁₂alkenyl, optionally substituted C₂-C₁₂alkynyl, optionally substituted C₂-C₁₂heteroalkyl, optionally substituted C₃-C₉cycloalkyl, optionally substituted C₃-C₉cycloalkenyl, optionally substituted C₂-C₁₂heterocycloalkyl, optionally substituted C₂-C₁₂heterocycloalkenyl, optionally substituted C₆-C₁₈aryl, optionally substituted C₁-C₁₈heteroaryl, optionally substituted C₃-C₉cycloalkylC₁-C₁₂alkyl, C₂-C₁₂heterocycloalkylC₁-C₁₂alkyl, optionally substituted C₆-C₁₈arylC₁-C₁₂alkyl, optionally substituted C₁-C₁₈heteroarylC₁-C₁₂ alkyl, optionally substituted C₆-C₁₈arylC₂-C₁₂heteroalkyl, optionally substituted C₃-C₉cycloalkylC₂-C₁₂heteroalkyl, optionally substituted C₆-C₁₈arylC₂-C₁₂heteroalkyl, optionally substituted C₂-C₁₂heterocycloalkylC₂-C₁₂heteroalkyl, and optionally substituted C₁-C₁₈heteroaryl C₂-C₁₂heteroalkyl, such that R¹ and R⁴ are not the same;
R² and R³ are each independently selected from the group consisting of: H, and C₁-C₁₂alkyl;
the method comprising reacting a compound of formula (II) 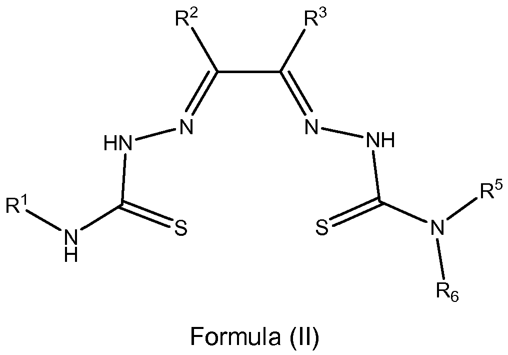
wherein R¹, R², and R³ are as defined above and R⁵ and R⁶ are each independently C₁-C₁₂alkyl, with a primary amine of formula (III) NH₂R⁴.

2. A method according to claim 1 wherein R¹ is methyl.

3. A method according to any one of claims 1 to 2 wherein R² and R³ are both methyl.

4. A method according to any one of claims 1 to 3 wherein R⁴ is a group of the formula:
-X-Y
wherein
X is selected from the group consisting of:
(a) a bond
(b) -(CH₂)ₘCO₂-
(c) -(CH₂)ₘCO-
(d) -(CH₂)ₘSO₃-
(e) -(CH₂)ₘSO₂-
(f) -(CH₂)mR⁸-
(g) -(CH₂)ₘCHR⁹R¹⁰;
(h) -(CH₂)ₘNHCO₂-
(i) -(CH₂)ₘNH-
(j) -(CH₂)ₘNR⁹-
(k) -(CH₂)ₘNHSO₂-
(l) -(CH₂)ₘSO₂-
(m) -(CH₂)ₘSO₃-
(n) -(CH₂)ₘR⁸-
(o) -(CH₂)ₘCHR⁹R¹⁰;
(p) -((CH₂)ₓO)_{y}-;
(q) -((CH₂)ₓNR₁₁)_{y}-;
wherein
m is an integer selected from the group consisting of 0, 1, 2, 3, 4, 5, 6, 7, 8, 9, and 10;
each x is independently an integer selected from the group consisting of 0, 1, 2, 3, 4, 5, 6, 7, 8, 9, and 10;
y is an integer selected from the group consisting of 0, 1, 2, 3, 4, 5, 6, 7, 8, 9, and 10;
R⁸ is selected from the group consisting of optionally substituted C₆-C₁₈aryl, and optionally substituted C₁-C₁₈heteroaryl,
each R⁹ and R¹⁰ is independently selected from the group consisting of CO₂H, optionally substituted C₁-C₁₂alkyl, and optionally substituted C₂-C₁₂heteroalkyl;
R¹¹ is independently selected from the group consisting of H, optionally substituted C₁-C₁₂alkyl, optionally substituted C₂-C₁₂heteroalkyl and a nitrogen protecting group; and
Y is selected from the group consisting of H, optionally substituted C₁-C₁₂alkyl, optionally substituted C₂-C₁₂alkenyl, optionally substituted C₂-C₁₂alkynyl, optionally substituted C₂-C₁₂heteroalkyl, optionally substituted C₃-C₉cycloalkyl, optionally substituted C₃-C₉cycloalkenyl, optionally substituted C₂-C₁₂heterocycloalkyl, optionally substituted C₂-C₁₂heterocycloalkenyl, optionally substituted C₆-C₁₈aryl, optionally substituted C₁-C₁₈heteroaryl, optionally substituted C₃-C₉cycloalkylC₁-C₁₂alkyl, C₂-C₁₂heterocycloalkylC₁-C₁₂alkyl, optionally substituted C₆-C₁₈arylC₁-C₁₂alkyl, optionally substituted C₁-C₁₈heteroarylC₁-C₁₂ alkyl, optionally substituted C₆-C₁₈arylC₂-C₁₂heteroalky, optionally substituted C₃-C₉cycloalkylC₂-C₁₂heteroalkyl, optionally substituted C₆-C₁₈arylC₂-C₁₂heteroalkyl, optionally substituted C₂-C₁₂heterocycloalkylC₂-C₁₂heteroalkyl, optionally substituted C₁-C₁₈heteroaryl C₂-C₁₂heteroalkyl, a peptide, a protein and a molecular recognition moiety.

5. A method according to claim 4 wherein Y is H or a molecular recognition moiety selected from the group consisting of antibodies, proteins, peptides, carbohydrates, nucleic acids, oligonucleotides, oligosaccharides and liposomes.

## Patentansprüche

1. Verfahren zur Herstellung einer Verbindung nach Formel (I): 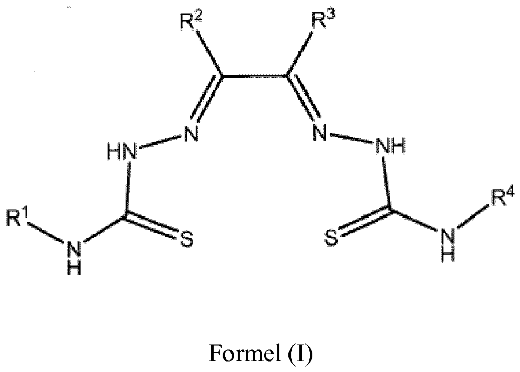 wobei
R¹ aus der Gruppe ausgewählt ist, die aus C₁-C₁₂Alkyl, C₂-C₁₂Alkenyl, C₂₋C₁₂Alkinyl, C₂-C₁₂Heteroalkyl, C₃-C₁₂Cycloalkyl, C₂-C₁₂Heterocycloalkyl, C₆-C₁₈Aryl und C₁-C₁₈Heteroaryl besteht;
R⁴ aus der Gruppe ausgewählt ist, die aus möglicherweise substituiertem C₁-C₁₂Alkyl, möglicherweise substituiertem C₂-C₁₂Alkenyl, möglicherweise substituiertem C₂-C₁₂Alkinyl, möglicherweise substituiertem C₂-C₁₂Heteroalkyl, möglicherweise substituiertem C₃-C₉Cycloalkyl, möglicherweise substituiertem C₃-C₉Cycloalkenyl, möglicherweise substituiertem C₂-C₁₂Heterocycloalkyl, möglicherweise substituiertem C₂-C₁₂Heterocycloalkenyl, möglicherweise substituiertem C₆-C₁₈Aryl, möglicherweise substituiertem C₁-C₁₈Heteroaryl, möglicherweise substituiertem C₃-C₉CycloalkylC₁-C₁₂alkyl, C₂-C₁₂HeterocycloalkylC₁-C₁₂alkyl, möglicherweise substituiertem C₆-C₁₈ArylC₁-C₁₂alkyl, möglicherweise substituiertem C₁-C₁₈HeteroarylC₁-C₁₂alkyl, möglicherweise substituiertem C₆-C₁₈ArylC₂-C₁₂heteroalkyl, möglicherweise substituiertem C₃-C₉CycloalkylC₂-C₁₂heteroalkyl, möglicherweise substituiertem C₆-C₁₈ArylC₂-C₁₂heteroalkyl, möglicherweise substituiertem C₂-C₁₂HeterocycloalkylC₂-C₁₂heteroalkyl und möglicherweise substituiertem C₁-C₁₈HeteroarylC₂-C₁₂heteroalkyl besteht, derart, dass R¹ und R⁴ nicht gleichartig sind;
R² und R³ unabhängig voneinander aus der Gruppe ausgewählt sind, die aus Folgendem besteht: H und C₁-C₁₂Alkyl;
wobei das Verfahren das Umsetzen einer Verbindung der Formel (II) 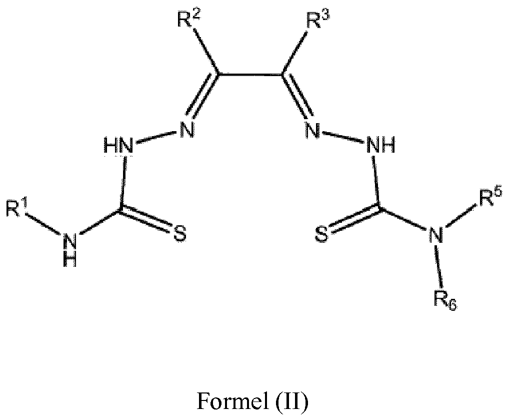
wobei R¹, R² und R³ den obigen Begriffsbestimmungen entsprechen und wobei R⁵ und R⁶ jeweils auf unabhängige Weise für C₁-C₁₂Alkyl stehen, mit einem primären Amin der Formel (III) NH₂R⁴ umfasst.

2. Verfahren nach Anspruch 1, wobei R¹ gleich Methyl ist.

3. Verfahren nach einem beliebigen der Ansprüche 1 bis 2, wobei R² und R³ beide gleich Methyl sind.

4. Verfahren nach einem beliebigen der Ansprüche 1 bis 3, wobei R⁴ für eine Gruppe nach der folgenden Formel steht:
-X-Y
wobei
X aus der Gruppe ausgewählt ist, die aus Folgendem besteht:
(a) einer Bindung
(b) -(CH₂)ₘCO₂-
(c) -(CH₂)ₘCO-
(d) -(CH₂)ₘSO₃-
(e) -(CH₂)ₘSO₂-
(f) -(CH₂)ₘR⁸-
(g) -(CH₂)ₘCHR⁹R¹⁰;
(h) -(CH₂)ₘNHCO₂-
(i) -(CH₂)ₘNH-
(j) -(CH₂)ₘNR⁹-
(k) -(CH₂)ₘNHSO₂-
(l) -(CH₂)ₘSO₂-
(m) -(CH₂)ₘSO;-
(n) -(CH₂)ₘR⁸-
(o) -(CH₂)ₘCHR⁹R¹⁰;
(p) -((CH₂)ₓO)_{y}-;
(q) -((CH₂))ₓNR₁₁)_{y}-;
wobei
m für eine ganze Zahl steht, die aus der Gruppe ausgewählt ist, welche aus 0, 1, 2, 3, 4, 5, 6, 7, 8, 9 und 10 besteht;
jedes x auf unabhängige Weise für eine ganze Zahl steht, die aus der Gruppe ausgewählt ist, welche aus 0, 1, 2, 3, 4, 5, 6, 7, 8, 9 und 10 besteht;
y für eine ganze Zahl steht, die aus der Gruppe ausgewählt ist, welche aus 0, 1, 2, 3, 4, 5, 6, 7, 8, 9 und 10 besteht;
R⁸ aus der Gruppe ausgewählt ist, die aus möglicherweise substituiertem C₆-C₁₈Aryl und möglicherweise substituiertem C₁-C₁₈Heteroaryl besteht,
jedes R⁹ und R¹⁰ auf unabhängige Weise aus der Gruppe ausgewählt ist, die aus CO₂H, möglicherweise substituiertem C₁-C₁₂Alkyl und möglicherweise substituiertem C₂-C₁₂Heteroalkyl besteht,
R¹¹ auf unabhängige Weise aus der Gruppe ausgewählt ist, die aus H, möglicherweise substituiertem C₁-C₁₂Alkyl, möglicherweise substituiertem C₂-C₁₂Heteroalkyl und einer stickstoffständigen Schutzgruppe besteht; und
Y aus der Gruppe ausgewählt ist, die aus H, möglicherweise substituiertem C₁-C₁₂Alkyl, möglicherweise substituiertem C₂-C₁₂Alkenyl, möglicherweise substituiertem C₂-C₁₂Alkinyl, möglicherweise substituiertem C₂-C₁₂Heteroalkyl, möglicherweise substituiertem C₃-C₉Cycloalkyl, möglicherweise substituiertem C₃-C₉Cycloalkenyl, möglicherweise substituiertem C₂-C₁₂Heterocycloalkyl, möglicherweise substituiertem C₂-C₁₂Heterocycloalkenyl, möglicherweise substituiertem C₆-C₁₈Aryl, möglicherweise substituiertem C₁-C₁₈Heteroaryl, möglicherweise substituiertem C₃-C₉CycloalkylC₁-C₁₂alkyl, C₂-C₁₂HeterocycloalkylC₁-C₁₂alkyl, möglicherweise substituiertem C₆-C₁₈ArylC₁-C₁₂alkyl, möglicherweise substituiertem C₁-C₁₈HeteroarylC₁-C₁₂alkyl, möglicherweise substituiertem C₆-C₁₈ArylC₂-C₁₂heteroalkyl, möglicherweise substituiertem C₃-C₉CycloalkylC₂-C₁₂heteroalkyl, möglicherweise substituiertem C₆-C₁₈ArylC₂-C₁₂heteroalkyl, möglicherweise substituiertem C₂-C₁₂HeterocycloalkylC₂-C₁₂heteroalkyl, möglicherweise substituiertem C₁-C₁₈HeteroarylC₂-C₁₂heteroalkyl, einem Peptid, einem Protein und einem Baustein, welcher der molekularen Erkennung dient, besteht.

5. Verfahren nach Anspruch 4, wobei Y für H steht, oder für einen Baustein, welcher der molekularen Erkennung dient und aus der Gruppe ausgewählt ist, die aus Antikörpern, Proteinen, Peptiden, Kohlenhydraten, Nukleinsäuren, Oligonukleotiden, Oligosacchariden und Liposomen besteht.

## Revendications

1. Procédé de préparation d'un composé de formule (I) : 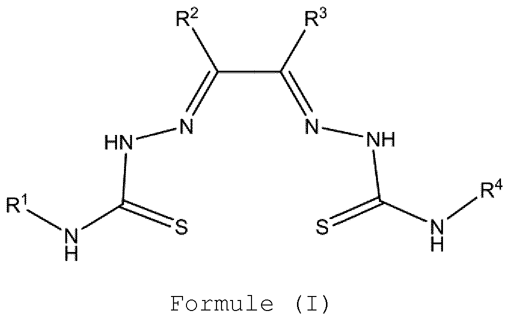 dans laquelle
R¹ est sélectionné dans le groupe constitué par les groupes alkyle en C₁ à C₁₂, alcényle en C₂ à C₁₂, alcynyle en C₂ à C₁₂, hétéroalkyle en C₂ à C₁₂, cycloalkyle en C₃ à C₁₂, hétérocycloalkyle en C₂ à C₁₂, aryle en C₆ à C₁₈ et hétéroaryle en C₁ à C₁₈ ;
R⁴ est sélectionné dans le groupe constitué par les groupes alkyle en C₁ à C₁₂ facultativement substitué, alcényle en C₂ à C₁₂ facultativement substitué, alcynyle en C₂ à C₁₂ facultativement substitué, hétéroalkyle en C₂ à C₁₂ facultativement substitué, cycloalkyle en C₃ à C₉ facultativement substitué, cycloalcényle en C₃ à C₉ facultativement substitué, hétérocycloalkyle en C₂ à C₁₂ facultativement substitué, hétérocycloalcényle en C₂ à C₁₂ facultativement substitué, aryle en C₆ à C₁₈ facultativement substitué, hétéroaryle en C₁ à C₁₈ facultativement substitué, (cycloalkyle en C₃ à C₉)alkyle en C₁ à C₁₂ facultativement substitué, (hétérocycloalkyle en C₂ à C₁₂) alkyle en C₁ à C₁₂ facultativement substitué, (aryle en C₆ à C₁₈)alkyle en C₁ à C₁₂ facultativement substitué, (hétéroaryle en C₁ à C₁₈) alkyle en C₁ à C₁₂ facultativement substitué, (aryle en C₆ à C₁₈)hétéroalkyle en C₂ à C₁₂ facultativement substitué, (cycloalkyle en C₃ à C₉)hétéroalkyle en C₂ à C₁₂ facultativement substitué, (aryle en C₆ à C₁₈) hétéroalkyle en C₂ à C₁₂ facultativement substitué, (hétérocycloalkyle en C₂ à C₁₂) hétéroalkyle en C₂ à C₁₂ facultativement substitué, et (hétéroaryle en C₁ à C₁₈) hétéroalkyle en C₂ à C₁₂ facultativement substitué, de sorte que R¹ et R⁴ ne soient pas identiques ;
R² et R³ sont chacun indépendamment sélectionnés dans le groupe constitué par H et un groupe alkyle en C₁ à C₁₂ ;
le procédé comprenant la réaction d'un composé de formule (II) : 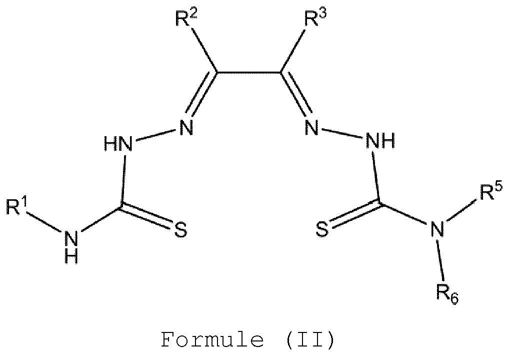
dans laquelle R¹, R² et R³ sont tels que définis ci-dessus et R⁵ et R⁶ sont chacun indépendamment un groupe alkyle en C₁ à C₁₂, avec une amine primaire de formule (III) NH₂R⁴.

2. Procédé selon la revendication 1, dans lequel R¹ est un groupe méthyle.

3. Procédé selon l'une quelconque des revendications 1 à 2, dans lequel R² et R³ sont tous les deux un groupe méthyle.

4. Procédé selon l'une quelconque des revendications 1 à 3, dans lequel R⁴ est un groupe de formule :
-X-Y
dans laquelle
X est sélectionné dans le groupe constitué par :
(a) une liaison
(b) -(CH₂)ₘCO₂-
(c) -(CH₂)ₘCO-
(d) -(CH₂)ₘSO₃-
(e) -(CH₂)ₘSO₂-
(f) -(CH₂)ₘR⁸-
(g) -(CH₂)ₘCHR⁹R¹⁰-
(h) -(CH₂)ₘNHCO₂-
(i) -(CH₂)ₘNH-
(j) - (CH₂)ₘNR⁹-
(k) -(CH₂)ₘNHSO₂-
(l) -(CH₂)ₘSO₂-
(m) -(CH₂)ₘSO₃-
(n) -(CH₂)ₘR⁸-
(o) -(CH₂)ₘCHR⁹R¹⁰-
(p) -((CH₂)ₓO)_{y}-
(q) -((CH₂)ₓNR₁₁)_{y}-
où
m est un nombre entier sélectionné dans le groupe constitué par 0, 1, 2, 3, 4, 5, 6, 7, 8, 9 et 10 ;
chaque x est indépendamment un nombre entier sélectionné dans le groupe constitué par 0, 1, 2, 3, 4, 5, 6, 7, 8, 9 et 10 ;
y est un nombre entier sélectionné dans le groupe constitué par 0, 1, 2, 3, 4, 5, 6, 7, 8, 9 et 10 ;
R⁸ est sélectionné dans le groupe constitué par les groupes aryle en C₆ à C₁₈ facultativement substitué et hétéroaryle en C₁ à C₁₈ facultativement substitué ;
chacun de R⁹ et R¹⁰ est indépendamment sélectionné dans le groupe constitué par les groupes CO₂H, alkyle en C₁ à C₁₂ facultativement substitué et hétéroalkyle en C₂ à C₁₂ facultativement substitué ;
R¹¹ est indépendamment sélectionné dans le groupe constitué par H, les groupes alkyle en C₁ à C₁₂ facultativement substitué, hétéroalkyle en C₂ à C₁₂ facultativement substitué et un groupe protégeant l'azote ; et
Y est sélectionné dans le groupe constitué par H, les groupes alkyle en C₁ à C₁₂ facultativement substitué, alcényle en C₂ à C₁₂ facultativement substitué, alcynyle en C₂ à C₁₂ facultativement substitué, hétéroalkyle en C₂ à C₁₂ facultativement substitué, cycloalkyle en C₃ à C₉ facultativement substitué, cycloalcényle en C₃ à C₉ facultativement substitué, hétérocycloalkyle en C₂ à C₁₂ facultativement substitué, hétérocycloalcényle en C₂ à C₁₂ facultativement substitué, aryle en C₆ à C₁₈ facultativement substitué, hétéroaryle en C₁ à C₁₈ facultativement substitué, (cycloalkyle en C₃ à C₉) alkyle en C₁ à C₁₂ facultativement substitué, (hétérocycloalkyle en C₂ à C₁₂) alkyle en C₁ à C₁₂ facultativement substitué, (aryle en C₆ à C₁₈)alkyle en C₁ à C₁₂ facultativement substitué, (hétéroaryle en C₁ à C₁₈)alkyle en C₁ à C₁₂ facultativement substitué, (aryle en C₆ à C₁₈) hétéroalkyle en C₂ à C₁₂ facultativement substitué, (cycloalkyle en C₃ à C₉) hétéroalkyle en C₂ à C₁₂ facultativement substitué, (aryle en C₆ à C₁₈) hétéroalkyle en C₂ à C₁₂ facultativement substitué, (hétérocycloalkyle en C₂ à C₁₂) hétéroalkyle en C₂ à C₁₂ facultativement substitué, (hétéroaryle en C₁ à C₁₈) hétéroalkyle en C₂ à C₁₂ facultativement substitué, un peptide, une protéine et un fragment de reconnaissance moléculaire.

5. Procédé selon la revendication 4, dans lequel Y est H ou un fragment de reconnaissance moléculaire sélectionné dans le groupe constitué par les anticorps, les protéines, les peptides, les glucides, les acides nucléiques, les oligonucléotides, les oligosaccharides et les liposomes.
